(19) Europäisches Patentamt

European Patent Office

Office européen des brevets

(11) Numéro de publication: **0 494 943 B1**

## (12) FASCICULE DE BREVET EUROPEEN

(45) Date de publication du fascicule du brevet: **30.08.95** (51) Int. Cl.⁶: **A61B 19/00**

(21) Numéro de dépôt: **90915319.9**

(22) Date de dépôt: **05.10.90**

(86) Numéro de dépôt internationale :
**PCT/FR90/00714**

(87) Numéro de publication internationale :
**WO 91/04711 (18.04.91 91/09)**

(54) **SYSTEME INTERACTIF D'INTERVENTION LOCALE A L'INTERIEUR D'UNE STRUCTURE NON HOMOGENE.**

(30) Priorité: **05.10.89 FR 8913028**

(43) Date de publication de la demande:
**22.07.92 Bulletin 92/30**

(45) Mention de la délivrance du brevet:
**30.08.95 Bulletin 95/35**

(84) Etats contractants désignés:
**AT BE CH DE DK ES FR GB IT LI LU NL SE**

(56) Documents cités:
**WO-A-88/09151**
**US-A- 4 638 798**

**IEEE Engineering in Medicine & Biology Society 11th Annual International Conference, 9-12 novembre 1989, Seattle, Washington, S. Lavallee: "A new system for computer assisted neurosurgery", pages 926-927**

(73) Titulaire: **DEEMED INTERNATIONAL**
**34/36 avenue de Friedland**
**F-75008 Paris (FR)**

(72) Inventeur: **HENRION, Joel**
**17, route de Châlons**
**F-51600 Suippes (FR)**
Inventeur: **SCRIBAN, Michel**
**72, chemin de Crapon**
**F-69360 Ternay (FR)**
Inventeur: **THIEBAUT, Jean-Baptiste**
**42, boulevard Saint-Marcel**
**F-75005 Paris (FR)**
Inventeur: **UHL, Jean-François**
**49, avenue de Versailles**
**F-75016 Paris (FR)**

Electronique Applications, no. 58, février-mars 1988, (Paris, FR), J. Trémolières: "Neurochirurgie assistée par ordinateur", pages 67-70

(74) Mandataire: **Pernez, Helga Breese Majerowicz, Conseils en Propriété Industrielle, CNIT World Trade Center 1, BP 434 F-92053 Paris la Défense (FR)**

## Description

L'invention concerne un système interactif d'intervention locale à l'intérieur d'une zone d'une structure non homogène.

L'exécution d'interventions locales à l'intérieur d'une structure non homogène, telles que des opérations de chirurgie intracranienne, chirurgie orthopédique, pose actuellement le problème de l'optimalisation du ou des trajets d'intervention afin d'assurer, d'une part, une intervention totale sur la zone ou structure d'intérêt, telle qu'une tumeur à traiter ou explorer, et, d'autre part, une lésion minimale des zones voisines ou connexes de la zone d'intérêt, ceci impliquant la localisation puis le choix des zones de la structure non homogène les moins sensibles à traverser ou les moins dommageables relativement à l'intégrité de la structure.

De nombreux travaux visant à apporter une solution au problème précité ont, jusqu'à ce jour, fait l'objet de publications. Parmi celles-ci, on peut citer l'article intitulé "Three Dimensional Digitizer (Neuro-navigator) : New Equipment for computed Tomography - Guided Stereotaxic Surgery" et publié par Eiju Watanabe, M.D. Takashi Watanabe, M.D., Shinya Manaka, M.D., Yoshiaki Mayanagi, M.D., et Kintomo Takakura, M.D Department of Neurosurgery, Faculty of Medicine, University of Tokyo, Japan dans la revue Surgery Neurol. 1987 : 27 p. 543-547 par Elsevier Science Publishing Co., Inc., et l'article "Neurochirurgie Assistée par Ordinateur", J. Trémolières, Revue Electronique Applications, n° 58, février-mars 1988, pages 67-70. Le brevet WO-A-88 09151 enseigne un équipement analogue.

Dans les publications précitées sont en particulier décrits un système et un mode opératoire à partir desquels un système de repérage de position tridimensionnelle, de type palpeur, permet de repérer les coordonnées de position tridimensionnelles d'une structure non homogène, telle que la tête d'un patient devant subir une intervention de neurochirurgie, puis de faire correspondre en fonction de la position relative de la structure non homogène une série d'images correspondantes constituées par des images en coupe bidimensionnelles selon une direction arbitraire, préalablement obtenues à l'aide d'un procédé d'imagerie médicale de type "scanner".

Le système et le mode opératoire précités présentent un intérêt certain pour le praticien intervenant puisque celui-ci dispose, au cours de l'intervention, outre la vue directe de l'intervention, d'au moins une vue en coupe bidimensionnelle lui permettant d'apprécier dans le plan de coupe la situation d'exécution de l'intervention.

Toutefois, et en raison de la conception même du système et du mode opératoire précités, ceux-ci ne permettent ni une représentation précise de la situation d'exécution de l'intervention, ni une conduite partiellement ou totalement automatisée de l'intervention selon un programme de cheminement de l'instrument déterminé préalablement à l'intervention.

Un tel système et un tel mode opératoire ne peuvent donc prétendre supprimer tout risque d'artefact puisque l'intervention reste conduite par le seul praticien.

On connaît également par l'article "Neurochirurgie assistée par ordinateur", Jacques Trémolières, Electronique Applications, n° 58, février-mars 1988, Paris p67-70, un système interactif selon le préambule de la revendication 1. Un tel système présente toutefois des inconvénients, et en particulier des risques d'erreurs dans la corrélation entre le référentiel du patient et celui des images affichées à l'écran. Ceci peut aboutir à des déviations importantes entre un trajet estimé à l'écran et le trajet réel dans la structure.

La présente invention a pour objet de remédier à ces inconvénients, et en particulier de proposer un système permettant une corrélation aussi exacte que possible, à tout instant, entre une modélisation d'intervention à l'écran et l'intervention proprement dite.

Un autre objet de la présente invention est également la mise en oeuvre d'un système permettant de simuler une trajectoire optimale de cheminement de l'outil, afin de constituer une intervention assistée ou entièrement programmée.

Enfin, un objet de la présente invention est de proposer un système permettant, à partir de le trajectoire simulée et de l'intervention programmée, d'asservir le déplacement de l'instrument ou outil à ladite trajectoire pour réaliser l'intervention programmée.

L'invention propose à cet effet un système interactif d'intervention locale à l'intérieur d'une zone d'une structure non homogène tel que défini dans la revendication 1.

Des aspects préférés de la présente invention sont exposés dans les sous-revendications.

Une description plus détaillée du système de l'invention sera donnée ci-après en référence aux dessins dans lesquels :
- la figure 1 représente une vue générale d'un système interactif d'intervention locale à l'intérieur d'une zone d'une structure non homogène selon la présente invention,

EP 0 494 943 B1

- la figure 2 représente, dans le cas où la structure non homogène est constituée par la tête d'un patient, et en vue d'une intervention de neurochirurgie, une structure de référence liée à la structure non homogène et permettant d'établir une corrélation entre un référentiel "patient" et un référentiel d'images du patient préalablement réalisées et mémorisées,

- la figure 3 représente un mode de réalisation avantageux de la distribution spatiale de la structure de référence de la figure 2,

- la figure 4 représente un mode de réalisation avantageux des moyens d'intervention installés sur une table d'opération dans le cas d'une intervention de neurochirurgie,

- la figure 5a et la figure 5b représentent un organigramme général d'étapes fonctionnelles mises en oeuvre par le système,

- les figures 7 et 8 représentent des organigrammes de programmes permettant la mise en oeuvre de certaines étapes fonctionnelles de la figure 5b,

- la figure 9a représente un organigramme d'un programme permettant la mise en oeuvre d'une étape fonctionnelle de la figure 5a,

- la figure 9b représente un organigramme d'un programme permettant la mise en oeuvre d'une autre étape fonctionnelle de la figure 5a,

- les figures 10a et 10b représentent un organigramme général des étapes successives d'un dialogue interactif entre le système de la présente invention et le praticien intervenant, et

- la figure 10c représente un organigramme général des étapes fonctionnelles successivement remplies par le système de l'invention, ayant l'intervention, préalablement à l'intervention, au cours de l'intervention et après l'intervention.

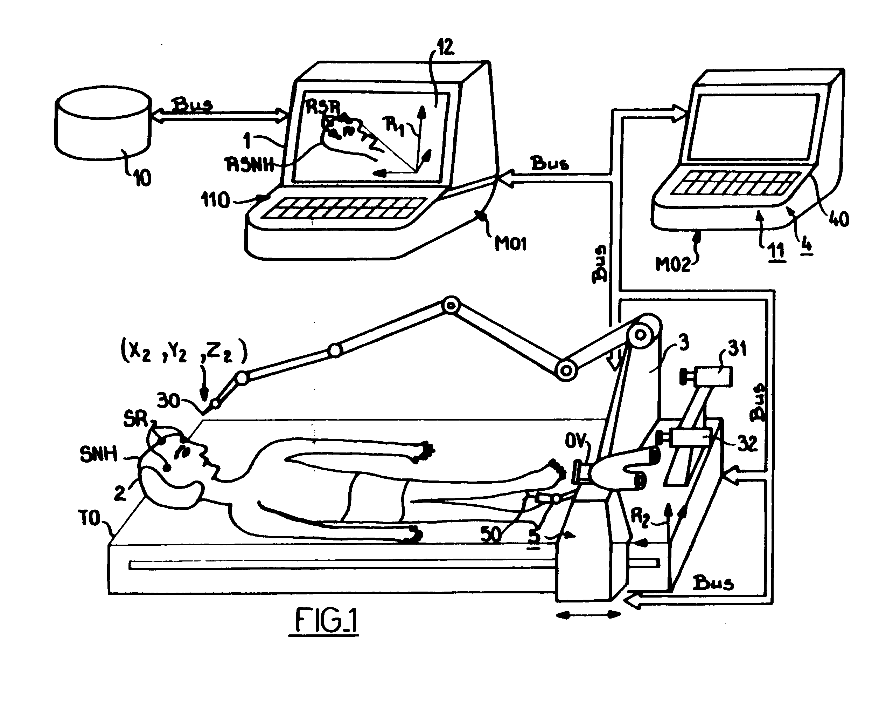

Le système interactif d'intervention locale selon l'invention sera tout d'abord décrit en liaison avec la figure 1.

Une structure non homogène, notée SNH, sur laquelle une intervention doit être effectuée, est constituée par exemple par la tête d'un patient dans laquelle une intervention de neurochirurgie doit être effectuée. Il est cependant entendu que le système de l'invention peut être utilisé afin de réaliser tout type d'intervention dans tout type de structure non homogène à l'intérieur de laquelle des unités ou éléments structurels et/ou fonctionnels peuvent être mis en évidence et dont l'intégrité, au cours de l'intervention, doit être respectée autant que possible.

Le système comprend des moyens notés 1 d'affichage dynamique en imagerie tridimensionnelle, par rapport à un premier référentiel $R_1$, d'une représentation (notée RSR) d'une structure de référence SR (décrite plus loin) liée à la structure SNH ainsi que d'une représentation ou modélisation de la structure non homogène notée RSNH.

Plus précisément, les moyens 1 permettent d'afficher une pluralité d'images tridimensionnelles successives, selon des angles différents, des représentations RSNH et RSR.

Le système de l'invention comporte également des moyens notés 2, de mise en position liée, par rapport à un deuxième référentiel $R_2$, des structures SNH et SR.

Dans le présent exemple non limitatif, la tête du patient, portant la structure de référence SR, est fixée sur une table d'opération TO sur laquelle les moyens 2 de mise en position liée sont fixés.

Bien entendu, le patient dont la tête a été installée dans les moyens 2 de mise en position liée a été soumis préalablement aux préparations habituelles, afin de lui permettre de subir l'intervention.

Les moyens 2 de mise en position liée par rapport à $R_2$ ne seront pas décrits en détail car ils peuvent être constitués par tout moyen (tel qu'un casque de maintien) normalement utilisé dans le domaine de la chirurgie ou de la neurochirurgie. Le référentiel $R_2$ petit arbitrairement être défini comme un trièdre trirectangle de référence lié à la table d'opération TO, ainsi que représenté en figure 1.

Des moyens 3 de repérage, par rapport au deuxième référentiel $R_2$, des coordonnées notées X2, Y2, Z2 de points quelconques, et en particulier d'un certain nombre de points de base de la structure de référence SR, sont en outre prévus.

Ces points de base constituant la structure de référence SR peuvent être constitués de certains points remarquables et/ou de marques apposées sur le patient, en des positions choisies par le praticien et notamment en ces points remarquables.

Le système de l'invention comprend en outre des moyens calculateurs 4 recevant des moyens 3 de repérage les coordonnées X2, X2, Z2.

Les moyens calculateurs 4, comme on le verra en détail plus loin, sont conçus pour élaborer des outils de transfert de référentiel optimaux en utilisant d'une part les coordonnées dans $R_2$, mesurées par le palpeur 3, d'une pluralité de points de base de la structure SR, et d'autre part les coordonnées dans $R_1$, déterminées par des outils graphiques du calculateur MO1 (pointage par souris, etc.), des images des points de base correspondants dans la représentation RSR, afin d'assurer la meilleure corrélation possible

4

entre les informations modélisées dans les équipements informatiques et les informations correspondantes de l'univers réel.

Il est en outre prévu des moyens 11 de transfert de référentiel conçus pour utiliser les outils ainsi élaborés et assurer en temps réel cette corrélation.

De plus, des moyens 40 sont prévus, comme on le verra en détail plus loin, pour déterminer ou modéliser une origine de référence d'intervention ORI et une direction d'intervention $\Delta$.

A l'aide des moyens 11, la direction modélisée d'intervention $\Delta$ peut, au moins préalablement à l'intervention et en début d'intervention, être matérialisée par un système de visée optique à la disposition du praticien, ce système de visée pouvant être asservi en position par rapport au deuxième référentiel $R_2$.

Le système de visée sera décrit ultérieurment.

Le système de la présente invention comporte enfin des moyens 5 d'intervention comportant un organe actif noté 50 dont la position est déterminée par rapport au deuxième référentiel $R_2$.

Selon une caractéristique avantageuse de l'invention, grâce aux moyens de transfert de référentiel 11, la position de l'organe actif peut être commandée dynamiquement à partir de la modélisation préalable de l'origine d'intervention ORI et de la direction d'intervention $\Delta$.

Les moyens 1 d'affichage dynamique en imagerie tridimensionnelle des représentations RSNH et RSR comportent un fichier 10 de données d'images bidimensionnelles. Le fichier 10 est par exemple constitué de données numérisées de coupes tomographiques, de radiographies, d'atlas de la tête du patient, et contenu dans une mémoire de masse appropriée.

Les coupes tomographiques successives peuvent être réalisées préalablement à l'intervention de manière classique, après que la structure de référence SR a été mis en place sur la structure non homogène SNH.

Selon un aspect avantageux, la structure de référence SR peut être constituée par une pluralité de marques ou de points remarquables qui soient à la fois palpables par les moyens de repérage 3 et détectables sur les images bidimensionnelles obtenues.

Bien entendu, les coupes tomographiques bidimensionnelles précitées peuvent également être réalisées par tout moyen d'imagerie médicale tel qu'un système à résonance magnétique nucléaire.

De façon caractéristique et bien connue, chaque image bidimensionnelle correspondant à une coupe tomographique de scanner correspond à une épaisseur de tranche de la structure d'environ 2 à 3 mm, les pixels ou éléments d'images dans le plan de ta coupe tomographique étant obtenus avec une précision de l'ordre de ± 1 mm. On comprend donc que les marques ou points constitutifs de la structure de référence SR apparaissent sur les images avec une incertitude de position, et un aspect important de l'invention va consister à minimiser ces incertitudes comme on le décrira plus loin.

Le système comporte également des premiers moyens 110 de calcul et de reconstitution d'images tridimensionnelles à partir des données du fichier 10.

Il comporte également un écran à haute résolution 12 permettant l'affichage d'une ou de plusieurs d'images tridimensionnelles ou bidimensionnelles constituant autant de représentations de la structure de référence RSR et de la structure non homogène SNH.

Avantageusement, les moyens de calcul 110, l'écran à haute résolution et la mémoire de masse contenant le fichier 10 font partie d'un calculateur du type station de travail de conception classique, noté MO1.

Préférentiellement, les premiers moyens de calcul 110 peuvent être constitués par un programme de type CAO implanté dans la station de travail MO1.

A titre d'exemple non limitatif, ce programme peut être dérivé du logiciel commercialisé sous la marque de commerce "AUTOCAD" par la société "AUTODESK" aux Etats-Unis d'Amérique.

Un tel logiciel permet, à partir des diverses images bidimensionnelles numérisées, de reconstituer des images tridimensionnelles constituant les représentations des structures RSR et RSNH selon des orientations quelconques.

Ainsi qu'on l'a en outre représenté en figure 1, les moyens de calcul 4 et 11 peuvent être constitués par un troisième calculateur noté MO2 sur la figure 1.

Les premier et second calculateurs MO1 et MO2 sont interconnectés par une liaison numérique classique (bus ou réseau).

En variante, les calculateurs MO1 et MO2 peuvent être remplacés par une station de travail unique.

Par exemple, les moyens 3 de repérage sont constitués par un palpeur tridimensionnel muni d'une pointe de touche 30.

Ce type de palpeur tridimensionnel connu en soi et non décrit en détail, est constitué d'une pluralité de bras articulés, repérés en position par rapport à une base solidaire de la table d'opération TO. Il permet de connaître les coordonnées de la pointe de touche 30 par rapport à l'origine $O_2$ du référentiel $R_2$ avec une

précision inférieure à 1 mm.

Le palpeur est par exemple muni de résolveurs délivrant des signaux représentatifs de la position instantanée de la pointe de touche 30 précitée. Les résolveurs sont eux-mêmes reliés à des circuits de conversion numérique/analogique et d'échantillonnage des valeurs représentatives de ces signaux, ces circuits d'échantillonnage étant interconnectés de manière classique au deuxième calculateur MO2 pour lui fournir les coordonnées X2, X2, Z2 de la pointe de touche 30.

En variante ou additionnellement, et de façon représentée schématiquement, les moyens de repérage 3 peuvent comprendre un jeu de caméras vidéo 31 et 32 (ou encore de caméras infra-rouges) permettant la prise de vue des structures SNH et SR.

Le jeu de caméras peut jouer le rôle d'un système stéréoscopique permettant le relevé de position des points de base de la structure de référence SR, ou d'autres points de la structure non homogène SNH, par rapport au deuxième référentiel $R_2$. Le relevé de position peut être effectué par exemple en ajoutant un système d'émission de faisceau laser permettant d'éclairer successivement les points dont on recherche les coordonnées, un logiciel approprié permettant de déterminer ensuite la position de ces points un à un par rapport à $R_2$. Ce logiciel ne sera pas décrit car il peut être constitué par des logiciels de reconnaissance de forme et de position normalement disponibles dans le commerce.

Selon un autre variante, les moyens de repérage 3 peuvent comprendre un système de télémétrie.

Dans ce cas, les marques de la structure SR peuvent être constituées par de petits radioémetteurs implantés par exemple sur les points considérés de la tête du patient et conçus pour être visibles sur les images bidimensionnelles, des capteurs optiques ou électromagnétiques appropriés (non représentés) étant prévus pour déterminer les coordonnées desdites marques dans le référentiel $R_2$ ou dans un référentiel lié à ce dernier.

Il est important de noter ici que la fonction générale des points de base de la structure de référence est d'une part d'être localisables individuellement sur la structure de référence, pour en déduire les coordonnées dans $R_2$, et d'autre part d'être visualisables sur les images bidimensionnelles afin d'être identifiées (par leurs coordonnées dans $R_1$) et incluses dans la représentation RSR à l'écran.

Il peut donc s'agir de marques spéciales apposées en des points quelconques de la surface latérale de structure SNH, ou encore en des points remarquables de celle-ci, ou encore, lorsque les points remarquables peuvent être localisés en eux-mêmes avec une bonne précision à la fois sur la structure SNH et sur les coupes 2D, des points remarquables totalement dépourvus de marques.

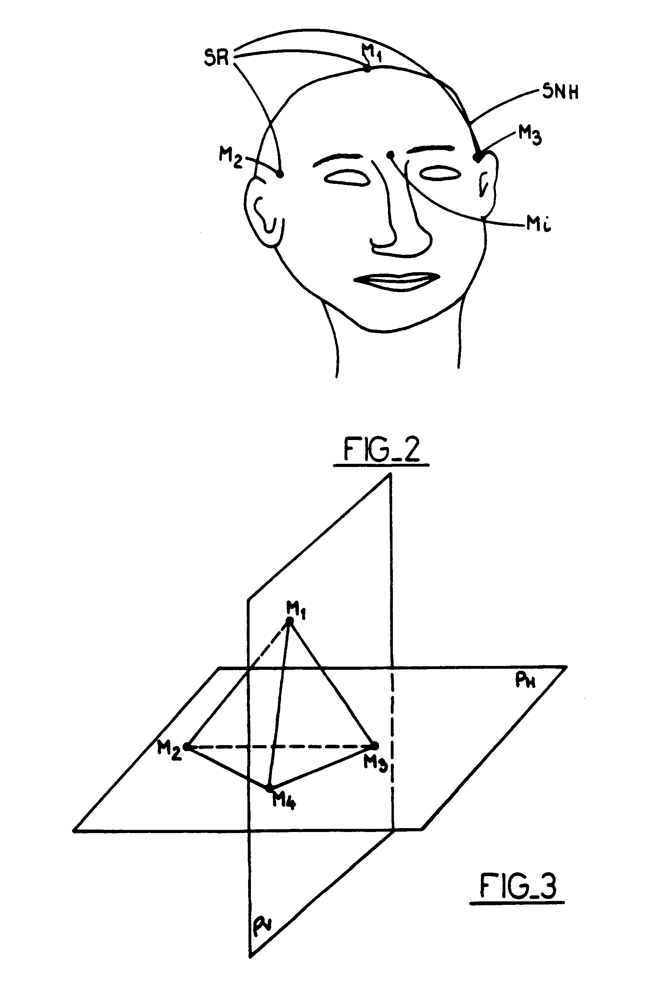

Sur la figure 2, on a représenté une pluralité de marques notées M1 à Mi, ces marques, dans le cas où la structure non homogène est constituée par la tête d'un patient, étant localisées par exemple entre les sourcils du patient, sur les tempes de ce dernier et au sommet du crâne en un point remarquable tel que le point frontal médian.

Plus généralement, pour un volume sensiblement ovoïde constituant la structure non homogène, on prévoit avantageusement quatre points de base au moins sur la surface extérieure du volume.

Ainsi qu'on l'a représenté en figure 3, les quatre marques M1 à M4 de la structure de référence sont distribuées de façon à définir de préférence un tétraèdre sensiblement symétrique. La symétrie du tétraèdre, représenté en figure 3, est matérialisée par le plan vertical de symétrie PV et le plan horizontal de symétrie PH.

Selon une caractéristique avantageuse, comme on le verra plus loin, les moyens pour élaborer les outils de transfert de référentiel sont conçus pour choisir trois points du tétraèdre qui vont définir le "meilleur plan" pour le transfert de référentiel.

Et la présence de quatre points ou davantage permet au(x) point(s) additionnel(s) de valider un choix déterminé.

Plus précisément, la présence de quatre points de base au minimum sur la structure de référence permet une recherche de la distorsion minimale entre les points saisis sur le patient par le moyen de repérage constitué par exemple par le palpeur tridimensionnel et les images de ces points sur la représentation en imagerie tridimensionnelle, dont les coordonnées sont calculées au cours du traitement. Le meilleur plan du tétraèdre précédemment décrit, c'est-à-dire le plan pour lequel l'incertitude sur les coordonnées des points entre les points effectivement saisis par le palpeur tridimensionnel et les points de la représentation de la structure de référence RSR, est minimale, devient alors le plan de référence pour le transfert de référentiel. On établira ainsi la meilleure corrélation entre une direction d'intervention modélisée et une origine d'intervention modélisée, d'une part, et l'action de l'organe 50. De préférence, l'origine d'intervention sera placée au centre de la zone dans laquelle l'intervention devra être effectuée, c'est-à-dire une tumeur observée ou traitée par exemple.

Par ailleurs, on pourra prendre en compte l'incertitude résiduelle constatée pour effectuer la représentation du modèle et des outils sur les moyens d'affichage dynamique.

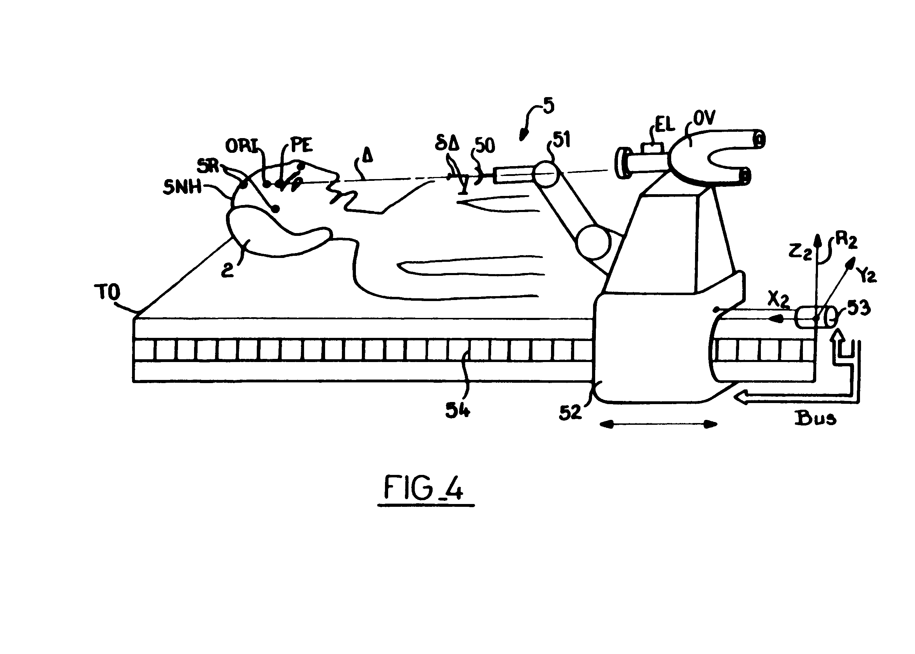

Une description plus détaillée des moyens d'intervention 5 sera maintenant donnée en liaison avec la figure 4.

De préférence, les moyens d'intervention 5 comportent un chariot 52 mobile en translation le long de la table d'opération TO, par exemple sur une crémaillère notée 54, en étant entraîné par un moteur non représenté, lui-même commandé via une liaison appropriée par le calculateur MO2 par exemple. Ce système de déplacement ne sera pas décrit en détail car il correspond à un système de déplacement classique disponible dans le commerce. En variante, le chariot 52 peut être mobile sur un trajet distinct et séparé de la table d'opération TO ou être immobile par rapport à la table d'opération et constituer alors un support.

Le chariot support 52 comporte en premier lieu un organe de visée OV, constituant le système de visée précédemment cité, lequel peut être constitué par une lunette binoculaire.

L'organe de visée OV permet au praticien, préalablement à l'intervention proprement-dite, ou pendant celle-ci, d'effectuer une visée de la position présumée de la zone dans laquelle l'intervention devra être effectuée.

En outre, et de manière non limitative, à l'organe de visée OV peut être associé un système d'émission laser hélium-néon, noté EL, permettant d'assurer le pointage d'un faisceau laser fin de positionnement ou de visée sur la structure SNH et notamment, comme on le verra en détail plus loin, d'indiquer au praticien la position d'un point d'entrée PE préalablement à l'intervention, pour permettre à celui-ci d'ouvrir le crâne à l'endroit approprié, et de lui indiquer également ce que sera la direction d'intervention. Accessoirement, l'éclairage du point considéré de la structure non homogène ou à tout le moins de la surface latérale de celle-ci permet aux caméras vidéo 31 et 32 d'effectuer, si nécessaire, un relevé de position.

De préférence, un système de mesure de position par télémétrie 53 est prévu pour assurer la mesure précise de la position du chariot 52 support de l'organe de visée OV et du système d'émission laser EL. Au cours de l'opération, et afin d'assurer l'intervention, le chariot 52 peut être déplacé le long de la crémaillère 54, la position du chariot 52 étant mesurée de manière très précise au moyen du système 53. Le système de télémétrie 53 est interconnecté par une liaison appropriée au microordinateur MO2.

Les moyens d'intervention 5 peuvent avantageusement être constitués par un bras guide 51 de l'organe actif 50.

Le bras guide 51 peut être avantageusement constitué par plusieurs tronçons articulés, chaque articulation étant munie de moteurs et, de résolveurs permettant d'assurer la commande de déplacement de l'extrémité du bras support et le relevé de position de cette même extrémité et donc de l'organe actif 50 selon six degrés de liberté par rapport au chariot 52. Les six degrés de liberté comprennent, bien entendu, trois degrés de liberté en translation par rapport à un référentiel lié au chariot 52 et trois degrés de liberté en rotation selon ces mêmes axes.

Ainsi le bras support 51 et l'organe 50 sont repérés en position instantanée par rapport au deuxième référentiel $R_2$, d'une part par l'intermédiaire du relevé de position du chariot mobile 52 et d'autre part par l'intermédiaire des résolveurs associés à chaque articulation du bras support 51.

Dans le cas d'une intervention chirurgicale intracranienne de neurochirurgie, l'organe actif 50 est amovible et peut être constitué par un outil de trépanation, une aiguille ou implant radioactif ou chimique, une tête d'émission laser ou de radioéléments ou un système de vision endoscopique. Ces différents organes ne seront pas décrits car ils correspondent à des instruments normalement utilisés en neurochirurgie.

La matérialisation de la direction d'intervention modélisée peut être effective au moyen de l'émetteur laser EL. Cette visée étant effectuée, le bras guide 51 peut alors être amené manuellement ou de façon asservie en superposition avec la direction d'intervention Δ.

Dans le cas d'un positionnement manuel, les résolveurs associés à l'organe de visée OV et à l'émetteur laser EL, le cas échéant, permettent de reporter le trajet de la direction de visée, constituant notamment la direction réelle d'intervention, sur la représentation de la structure non homogène dans les moyens d'affichage dynamique 1.

En outre, ainsi qu'il sera décrit ultérieurement et de manière préférentielle, le praticien intervenant pourra tout d'abord définir un trajet d'intervention simulé et y asservir les déplacements de l'organe actif 50 dans la structure non homogène pour assurer effectivement tout ou partie de l'intervention.

Dans ce cas, la progression de l'outil d'intervention 50 est alors directement asservie au trajet simulé (données ORI, Δ) en faisant intervenir les moyens 11 de transfert de référentiel pour exprimer le trajet dans le référentiel $R_2$.

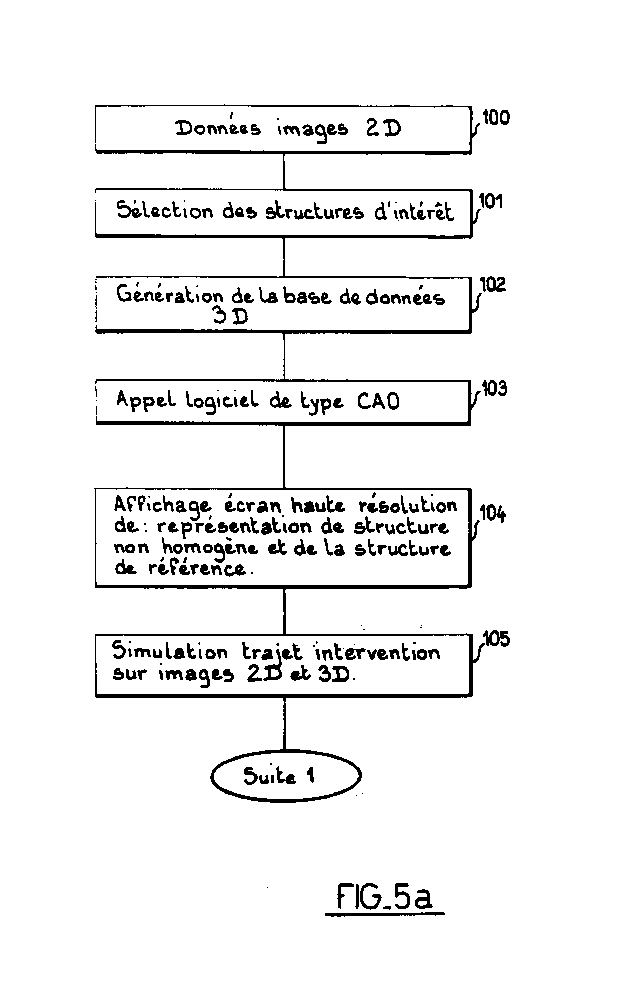

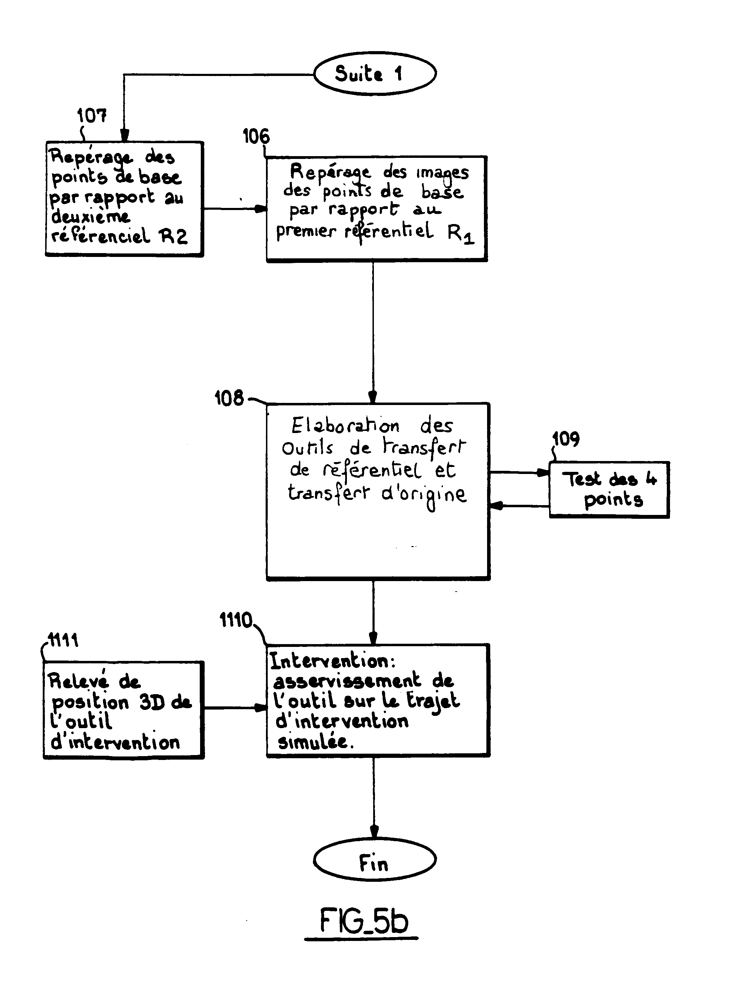

Une description plus détaillée de la mise en oeuvre et du mode opératoire du système de l'invention, sera maintenant décrite en liaison avec les figures 5a et 5b.

Selon la figure 5a, la première étape consiste à obtenir et organiser en mémoire les données d'images bidimensionnelles (étape 100). Tout d'abord, la structure non homogène SNH est préparée. Dans le cas d'une intervention de neurochirurgie par exemple, cela signifie que la tête du patient peut être munie des marques constituant les points de base de la structure de référence SR. Ces marques peuvent être réalisées au moyen de points constitués par un colorant absorbant partiellement les rayons X, tel qu'un colorant radio-opaque.

Les marques précitées sont implantées par le praticien sur la tête du patient en des points remarquables de celles-ci et la structure non homogène SNH peut alors être soumise à une prise d'images par tomographie par exemple, au moyen d'un appareil de type scanner à rayons X.

Cette opération ne sera pas décrite en détail car elle correspond à des opérations classiques dans le domaine de l'imagerie médicale.

Les données d'images bidimensionnelles obtenues sont alors constituées en données numérisées dans le fichier 10, ces données étant elles-mêmes repérées par rapport au référentiel $R_1$ et permettant de restituer à la demande les images bidimensionnelles sur les moyens d'affichage dynamique 1, ces images représentant des coupes superposées de la structure non homogène SNH.

A partir des données d'images numérisées à la disposition du praticien, ce dernier procède alors, comme indiqué en 101 en figure 5a, à la sélection des structures d'intérêt des images précitées.

Le but de cette étape est de faciliter le travail du praticien en formant des images tridimensionnelles qui ne comportent que les contours des éléments de la structure qui sont essentiels pour la définition géométrique et le contrôle en temps réel du déroulement de l'intervention.

Dans le cas où la structure non homogène SNH est constituée par la tête d'un patient, une analyse des données d'images bidimensionnelles permet, à partir de valeurs de densité optique des points-images correspondants, d'extraire d'emblée les contours du crâne, de vérifier les échelles de distance, etc...

De préférence, les opérations précitées sont effectuées sur un rectangle d'intérêt pour une image bidimensionnelle donnée, ce qui permet, par déplacement du rectangle d'intérêt, de couvrir l'ensemble de l'image.

L'analyse ci-dessus est effectuée au moyen d'un logiciel adapté qui permet ainsi d'extraire et de vectoriser les contours des structures qui seront modélisées dans les représentations RSNH et RSR.

Les structures modélisées dans le cas d'une intervention de neurochirurgie sont par exemple le crâne, les ventricules cérébraux, la tumeur devant être observée ou traitée, la faux du cerveau ainsi que les diverses zones fonctionnelles.

Selon un aspect du système interactif de l'invention, le praticien peut disposer d'une table à digitaliser ou autre périphérique graphique permettant, pour chaque image bidimensionnelle affichée, de rectifier ou compléter la définition du contour d'une zone d'intérêt particulière.

On notera enfin qu'en superposant les contours extraits à l'image bidimensionnelle affichée, le praticien pourra valider les extractions réalisées.

Les contours extraits sont ensuite traités par échantillonnage de points pour obtenir leurs coordonnées dans le référentiel $R_1$, ces coordonnées pouvant être constituées en un fichier de type ASCII. Il s'agit de l'étape 102 de génération de la base de données tridimensionnelles.

Cette étape est suivie d'une étape 103 de reconstruction du modèle tridimensionnel. Cette étape consiste tout d'abord, à l'aide du logiciel de type CAO, à réaliser à partir des contours des structures d'intérêt constituées en images bidimensionnelles vectorisées une extrapolation entre les différents plans de coupe.

L'extrapolation présitée est réalisée de préférence au moyen d'un algorithme de type "B-Spline" qui apparaît le mieux adapté. Cette extrapolation transforme une information discrète, à savoir les coupes successives obtenues au moyen de l'analyse scanner, en un modèle continu permettant la représentation tridimensionnelle des enveloppes volumiques des structures.

Il est à noter que la reconstruction des volumes constituant les structures d'intérêt introduit une approximation liée notamment à l'espacement et à l'épaisseur non nulle des coupes d'acquisition. Une caractéristique importante de l'invention, comme expliqué en détail par ailleurs, est d'une part de minimiser les incertitudes résultantes dans la corrélation patient-modèle, et d'autre part de prendre en compte les incertitudes résiduelles.

Le logiciel de type CAO utilisé possède des fonctions standard qui permettent de manipuler le modèle dans l'espace en l'affichant selon des points de vue différents sur seul critère défini par le praticien (étape 104).

Le logiciel peut également reconstruire des plans de coupe de représentation de la structure non homogène différents des plans des images du fichier 10, ce qui permet notamment de développer des connaissances enrichissant les données de la représentation par constitution d'un atlas neuro-anatomique.

EP 0 494 943 B1

Le praticien peut ensuite (étape 105) déterminer un modèle de stratégie d'intervention en tenant compte des structures d'intérêt modélisées, en évaluant des rapports de distances et d'angles sur les représentations bi- et tridimensionnelles affichées.

Cette stratégie d'intervention va consister, concrètement, d'une part à localiser la tumeur et à y associer un "point cible", qui pourra ensuite tenir lieu d'origine commune de tous les objets (réels et images) traités par le système, et d'autre part à déterminer un trajet simulé d'intervention respectant au maximum l'intégrité des structures d'intérêt. Cette étape peut être réalisée "au bureau", en ne faisant intervenir que la station de travail.

Une fois cette opération effectuée, et préalablement à l'intervention, la phase suivante consiste à mettre en oeuvre les étapes nécessaires à l'établissement d'une corrélation aussi exacte que possible entre la structure SNH (univers réel) et la représentation RSNH (univers informatique). Il s'agit des étapes 106 à 109 de la figure 5b.

Tout d'abord, ainsi que représenté en figure 5b à l'étape 107, on procède repérage des points de base de la structure de référence SR par rapport au deuxième référentiel à l'aide des moyens de repérage 3, en délivrant au système les coordonnées X2, Y2, Z2 desdits points de base.

L'étape suivante 106 consiste à identifier sur les représentations RSNH et RSR affichées à l'écran les images des points de base que l'on vient de repérer. Plus précisément, à l'aide de périphériques graphiques appropriés, on sélectionne une à une ces représentations (images) des points de base, et la station de travail fournit à chaque fois (en l'espèce au calculateur M02) les coordonnées de ces points représentés dans le référentiel $R_1$.

Ainsi le calculateur M02 dispose d'un premier jeu de coordonnées tridimensionnelles, représentatives de la position des points de base dans $R_2$, et d'un second jeu de coordonnées tridimensionnelles, représentatives de la position des représentations des points de base dans $R_1$.

Selon un aspect essentiel de l'invention, on va utiliser ces données pour élaborer en 108, 109 des outils de transfert de référentiel (de $R_1$ vers $R_2$ et réciproquement) en faisant appel à un référentiel intermédiaire déterminé à partir des points de base et constituant un référentiel intermédiaire propre au modèle reconstruit.

Plus précisément, on construit le référentiel intermédiaire à partir de trois points de base choisis de telle sorte que, dans ce référentiel, les coordonnées des autres points de bases après transfert depuis $R_2$ et les coordonnées des représentations de ces autres points de base après transfert depuis $R_1$ s'expriment avec la plus grande cohérence et la distorsion minimale.

Lorsque l'étape d'élaboration des outils de transfert de référentiels est achevée, ces outils peuvent être utilisés par le système pour assurer le couplage optimal entre l'univers réel et l'univers informatique (étape 1110).

Par ailleurs, selon un aspect auxiliaire de la présente invention, le système peut créer sur les moyens d'affichage une représentation de la structure non homgène et de l'organe d'intervention qui tienne compte des écarts et distortions subsistant après que les "meilleurs" outils de transfert de référentiel aient été sélectionnés (incertitudes résiduelles). Plus précisément, de' ces écarts peut être déduite par les moyens de calcul une erreur type susceptible d'apparaître dans le positionnement mutuel entre la représentation de la structure non homogène et la représentation d'éléments (outils, axes de visée, etc...) référencés sur $R_2$ lors de l'utilisation des outils de transfert de référentiel. Cette incertitude résiduelle, qui peut être dans la pratique concrétisée par une matrice d'erreur, peut être utilisée par exemple pour représenter certains contours (outil, structures d'intérêt à éviter lors de l'intervention, etc...) avec des dimensions plus grandes que celles qui seraient normalement représentées à partir des données de la base tridimensionnelle ou à l'aide de coordonnées repérées dans $R_2$, lesdites dimensions plus grandes étant déduites des dimensions "normales" en faisant intervenir la matrice d'erreur. Par exemple, si l'organe était représenté normalement, en section transversale, par un cercle de diamètre D1, on peut représenter concrètement un cercle de diamètre D2>D1, avec la différence D2-D1 déduite de la valeur d'erreur type. De cette manière, lorsqu'on choisira une direction d'intervention permettant d'éviter de traverser certaines structures d'intérêt, la prise en compte d'une taille "agrandie" de l'outil d'intervention supprimera tout risque qu'à cause des erreurs précitées, l'organe traverse accidentellement ces structures.

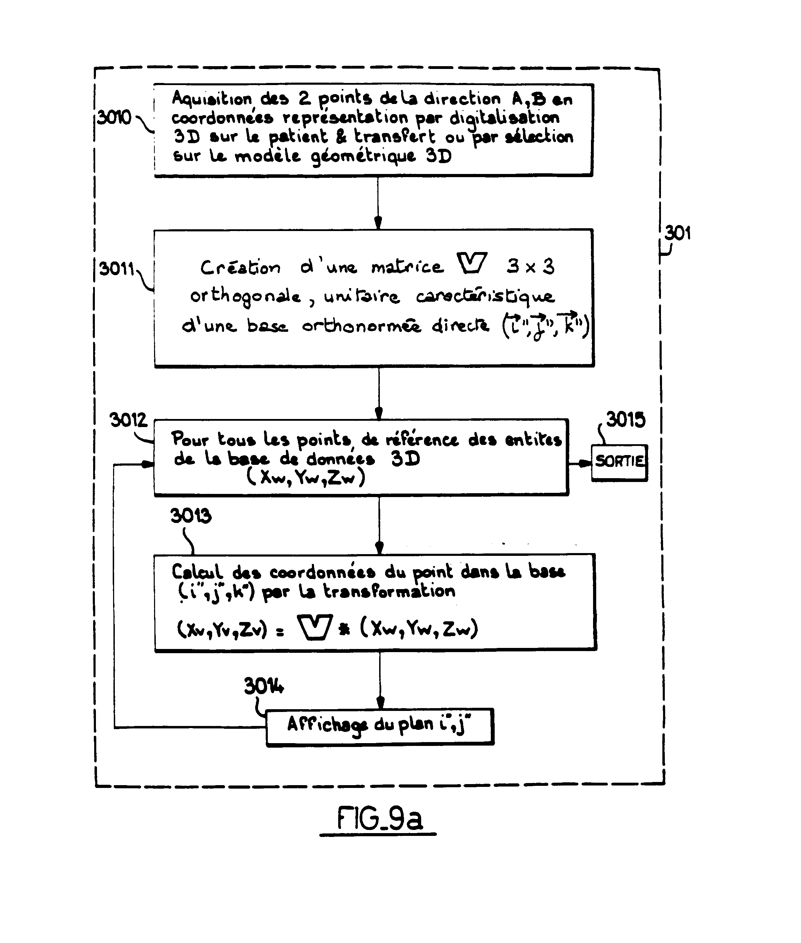

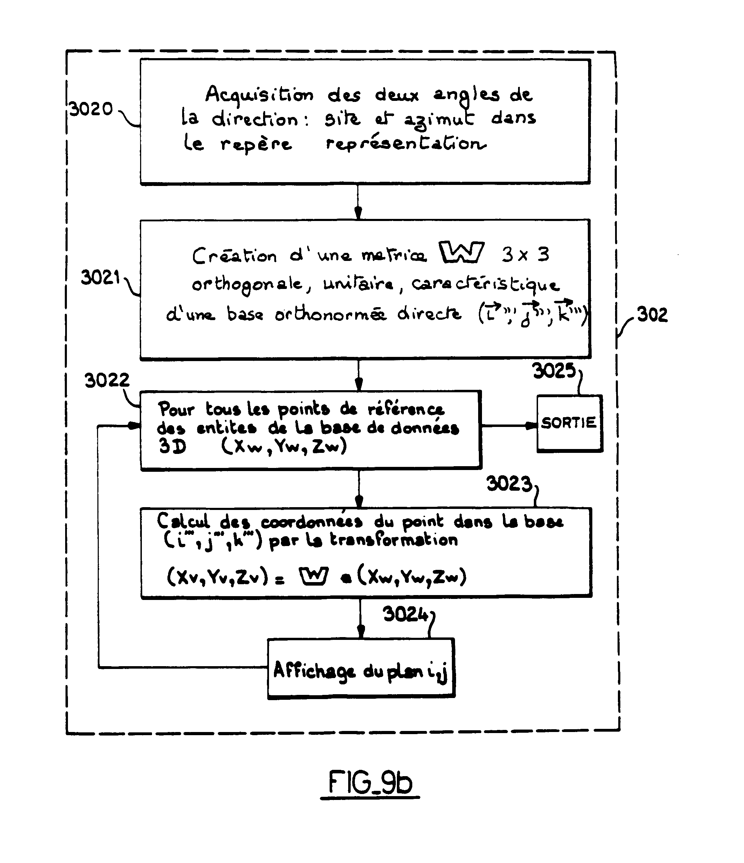

De retour à l'étape 105, et comme on le verra plus en détail en référence aux figures 9a et 9b, l'origine de référence d'intervention ORI et la direction d'intervention Δ, c'est-à-dire le trajet simulé d'intervention, peuvent être déterminées selon différentes procédures.

Selon une première procédure, la trajectoire peut être définie à partir de deux points, à savoir un point d'entrée PE (figure 3) et un point cible, c'est-à-dire sensiblement le centre de la structure d'intérêt constituée par la tumeur à observer ou traiter. Initialement, ces deux points sont localisés sur le modèle représenté à l'écran.

9

Selon une deuxième modalité, la trajectoire peut être déterminée à partir du point cible précité et d'une direction qui tient compte des types de structures d'intérêt et de leurs positions en vue de respecter optimalement leur intégrité.

Suite à l'étape 108 précitée, le praticien peut effectuer à l'étape 1110 l'intervention proprement-dite.

L'intervention peut être avantageusement effectuée par asservissement de l'outil ou organe actif sur le trajet d'intervention simulé, déterminé à l'étape 1110.

En variante, étant donné que le bras support 51 de l'organe actif, muni de ses résolveurs, délivre en continu au système les coordonnées dans $R_2$ dudit organe actif, il est également possible d'effectuer l'opération manuellement ou semi-manuellement, en contrôlant sur l'écran la position et les mouvements d'une représentation de l'outil et en les confrontant au trajet d'intervention simulé, affiché.

On notera en outre que la direction d'intervention modélisée peut être matérialisée à l'aide du faisceau laser décrit précédemment, le positionnement de ce dernier (par rapport à $R_2$) étant de même réalisé grâce aux outils de transfert de référentiel.

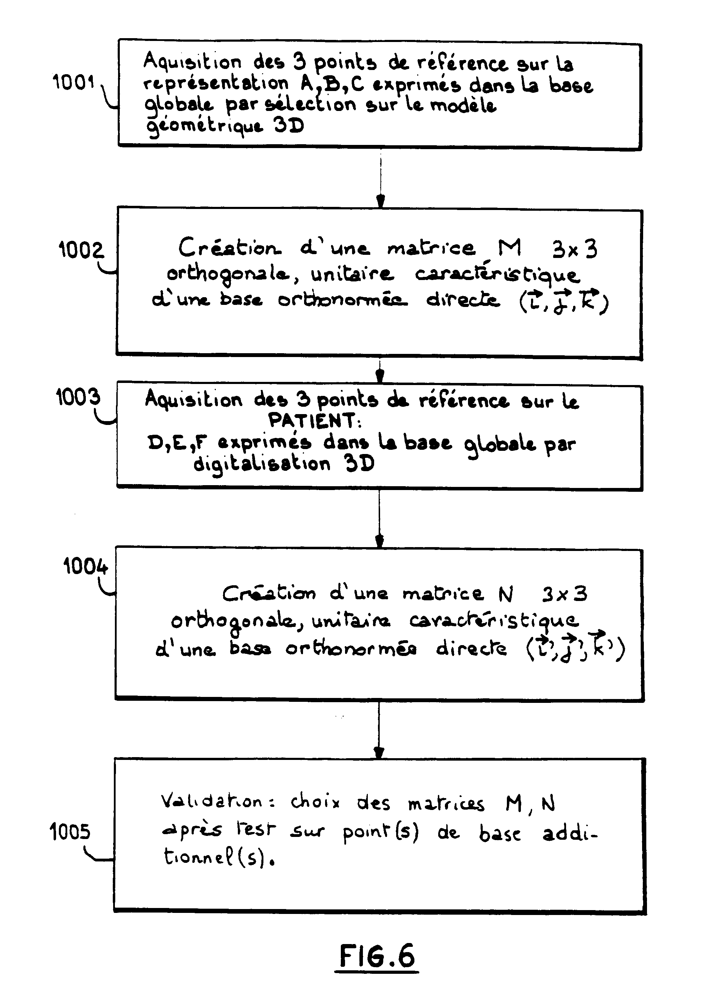

On va maintenant décrire plus en détail en référence aux figures 6, 7, 8, 9a et 9b certaines fonctionnalités du système de l'invention.

On décrira tout d'abord en référence à la figure 6 le module d'élaboration des outils de transfert de référentiel (étapes 108, 109 de la figure 5b).

Ce module comprend un premier sous-module 1001 d'acquisition de trois points A, B, C, images des points de base de SR, sur la représentation RSNH (les coordonnées de ces points étant exprimées dans le référentiel informatique $R_1$), par sélections successives de ces points sur la représentation. A cet effet, le praticien est amené, au moyen d'une interface graphique tel qu'une "souris", à pointer successivement les trois points A,B,C choisis.

Le module de préparation des outils de transfert comporte également un deuxième sous-module, noté 1002, de création d'une matrice M orthogonale tridimensionnelle unitaire, cette matrice étant caractéristique d'une base orthonormée directe représentée par trois vecteurs unitaires $\vec{i}$, $\vec{j}$, $\vec{k}$, lesquels définissent un référentiel intermédiaire lié à $R_1$.

Les vecteurs unitaires $\vec{i}$, $\vec{j}$ et $\vec{k}$ sont donnés par les relations :

$$\vec{j} = \vec{AB}/\|\vec{AB}\|$$
$$\vec{k} = (\vec{BA} \wedge \vec{BC})/\|\vec{BA} \wedge \vec{BC}\|$$
$$\vec{i} = \vec{j} \wedge \vec{k}$$

où $\|\ \|$ désigne la norme du vecteur considéré.

Dans les relations précitées, le signe "$\wedge$" désigne le produit vectoriel des vecteurs considérés.

De la même manière, le module de préparation des outils de transfert comporte un troisième sous-module noté 1003 d'acquisition de trois points de base D, E, F, de la structure SR, ces trois points étant ceux dont les images sur le modèle sont respectivement les points A,B,C. Dans ce but, le praticien, par exemple au moyen de la pointe de touche 30, palpe successivement ces trois points pour obtenir leurs coordonnées dans $R_2$.

Le sous-module 1003 est lui-même suivi, ainsi que représenté en figure 6, par un quatrième sous-module 1004 de création d'une matrice N orthogonale tridimensionnelle unitaire, caractéristique d'une base orthonormée directe comportant trois vecteurs unitaires $\vec{i'}$, $\vec{j'}$, $\vec{k'}$ et liée au deuxième référentiel $R_2$ du fait que la structure non homogène SNH est en position liée par rapport à ce référentiel.

Les trois vecteurs unitaires $\vec{i'}$, $\vec{j'}$, $\vec{k'}$ sont définis par les relations :

$$\vec{j'} = \vec{DE}/\|\vec{DE}\|$$
$$\vec{k'} = (\vec{ED} \wedge \vec{EF})/\|\vec{ED} \wedge \vec{EF}\|$$
$$\vec{i'} = \vec{j'} \wedge \vec{k'}$$

Comme indiqué plus haut, autant les points de base de la structure de référence peuvent être repérés dans $R_2$ avec une bonne précision, autant leur représentation dans la base informatique $R_1$ est repérée avec une certaine marge d'erreur compte tenu d'une part de l'épaisseur non nulle (typiquement de 2 à 3 mm) des tranches représentées par les images bidimensionnelles du fichier 10, et d'autre part (en général dans une moindre mesure) de la définition de chaque élément d'image ou pixel d'une coupe.

Selon l'invention, une fois qu'une paire de matrices de transfert M, N a été élaborée avec des points A, B, C, D, E, F choisis, on cherche à valider ce choix en utilisant un ou plusieurs points de base additionnels; plus précisément, pour le ou chaque point de base additionnel, on repère ce point dans $R_2$ à l'aide du palpeur 30, on repère la représentation de ce point dans $R_1$ après sélection sur l'écran, puis on applique aux coordonnées obtenues les matrices N et M, respectivement, pour obtenir leurs expressions respectivement dans les bases $(\vec{i'}, \vec{j'}, \vec{k'})$ et $(\vec{i}, \vec{j}, \vec{k})$. Si ces expressions concordent bien, on peut assimiler ces deux bases à un même référentiel intermédiaire, ce qui assure le couplage mathématique aussi exact que possible entre le référentiel informatique $R_1$ lié au modèle et le référentiel "réel" $R_2$ lié au patient.

En pratique, le module d'élaboration des outils de transfert de référentiel peut être conçu pour effectuer successivement les étapes 1001 à 1004 sur des triplets de base à chaque fois différents (par exemple, si l'on a défini quatre points de base, associés à quatre représentations dans RSR, il existe quatre triplets possibles), pour effectuer l'étape de validation 1005 pour chacun de ces choix et enfin pour choisir le triplet pour lequel la meilleure validation est obtenue, c'est-à-dire pour lequel l'écart entre les expressions précitées est le plus faible. Ce triplet définit le "meilleur plan" mentionné par ailleurs dans la description, et aboutit aux "meilleures" matrices de transfert M et N.

En variante, le choix du meilleur plan pourra être effectué au moins en partie par le praticien grâce à son expérience.

Il est à noter que le transfert de référentiel ne sera achevé qu'en complétant le calcul matriciel avec les matrices M, N avec un transfert d'origine, de façon à créer une nouvelle origine commune par exemple au centre de la tumeur à observer ou à traiter (point ORI). Ce transfert d'origine est effectué simplement par soustraction appropriées de vecteurs d'une part sur les coordonnées dans $R_1$, d'autre part sur les coordonnées dans $R_2$. ces vecteurs à soustraire sont déterminés après localisation du centre de la tumeur sur la représentation.

Par ailleurs, les moyens décrits ci-dessus pour établir le couplage entre l'univers du patient et l'univers du modèle peuvent être également utilisés pour coupler à l'univers du modèle celui de données d'atlas, également mémorisées dans la station de travail et exprimées dans un référentiel différent, noté $R_3$. Dans ce cas, puisque ces données ne comportent aucune marque spécifique visible, l'élaboration de matrices décrite plus haut est effectuée en substituant à ces marques les positions de points remarquables de la tête du patient. Il peut s'agir de points temporaux, point frontal médian, sommet du crâne, centre de gravité des orbites des yeux...

Les points correspondants du modèle peuvent être obtenus soit par sélection par souris ou tablette graphique sur le modèle, soit par palpage sur le patient lui-même puis utilisation des matrices de transfert.

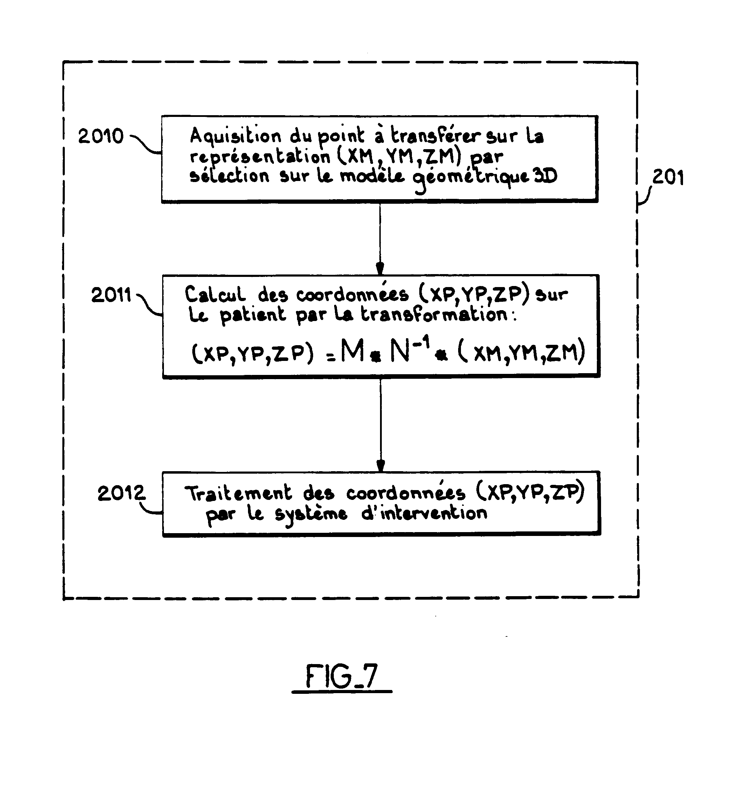

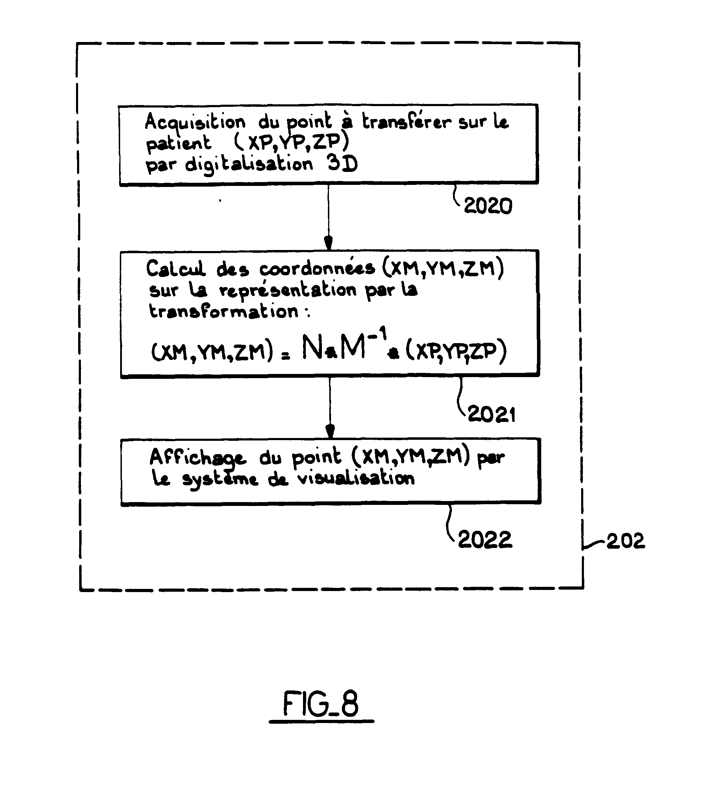

L'étape ci-dessus d'élaboration des outils de transfert ou référentiel, conduite en pratique par les moyens de calcul 4, permet subséquemment de mettre en oeuvre les moyens de transfert de référentiel (figs. 7 et 8).

En référence à la figure 7, le premier sous-module 201 de transfert comporte une procédure notée 2010 d'acquisition des coordonnées XM, YM, ZM, exprimées dans $R_1$, du point à transférer, par sélection sur la représentation.

La procédure 2010 suivie d'une procédure 2011 de calcul des coordonnées XP, YP, ZP (exprimées dans $R_2$) du point réel correspondant sur le patient par la transformation :

$$\{XP, YP, ZP\} = M * N^{-1} * \{XM, YM, ZM\}$$

où $M * N^{-1}$ représente le produit de la matrice M et de la matrice N inverse.

La procédure 2011 est suivie d'une procédure 2012 de traitement en utilisant les coordonnées calculées XP, YP, ZP, par exemple pour indiquer le point correspondant sur la surface de la structure SNH au moyen du système d'émission laser EL, ou encore pour assurer l'intervention au point considéré de coordonnées XP, YP, ZP (par asservissement de l'organe actif).

Inversement, afin d'assurer un transfert de SNH vers RSNH, le deuxième sous-module 202 comprend (figure 8) une procédure notée 2020 d'acquisition sur la structure SNH des coordonnées XP, YP, ZP (exprimées dans $R_2$) d'un point à transférer.

Ces coordonnées peuvent être obtenues au moyen de la pointe de touche 30 par exemple. La procédure 2020 est suivie d'une procédure 2021 de calcul des coordonnées correspondantes XM, YM, ZM dans $R_1$ par la transformation:

$$\{XM, YM, ZM\} = N * M^{-1} * \{XP, YP, ZP\}$$

Une procédure 2022 permet ensuite d'effectuer l'affichage du point de coordonnées XM, YM, ZM sur le modèle ou encore d'une droite ou d'un plan passant par ce point et répondant par ailleurs à d'autres critères.

On notera ici que les deux sous-modules 201, 202 peuvent utilisés par le praticien à tout instant dans le but de vérifier le caractère valide des outils de transfert; en particulier, on peut vérifier à tout moment qu'un point de base réel, de coordonnées connues à la fois dans $R_2$ et dans $R_1$ (par exemple un point de base de SR ou un point remarquable quelconque de la structure SNH visible sur les images), se replace bien par rapport à son image après transfert de coordonnées à l'étape 2011.

En cas de différence excessive, on effectue une nouvelle étape d'élaboration des outils de transfert.

Par ailleurs, les sous-modules 201, 202 peuvent être conçus pour intégrer également la prise en compte de l'incertitude résiduelle, comme évoqué plus haut, par exemple afin de représenter sur l'écran un point palpé non pas de façon ponctuelle, mais par exemple sous forme d'un cercle ou une sphère représentatif de ladite incertitude.

A partir d'un trajet d'intervention simulé, par exemple sur la représentation RSNH, ou à partir de toute autre droite choisie par le praticien, l'invention permet par ailleurs de représenter le modèle sur l'écran selon un point de vue correspondant à cette droite. Ainsi le troisième sous-programme de transfert comporte, comme représenté en figures 9a et 9b, un premier module 301 de visualisation de la représentation dans une direction donnée par deux points et un deuxième module 302 de visualisation de la représentation dans une direction donnée par un angle de site et un angle d'azimut.

Le premier module 301 de visualisation de la représentation dans une direction donnée par deux points comporte un premier sous-module noté 3010 permettant l'acquisition des deux points considérés qui vont définir la direction choisie. Les coordonnées de ces points sont exprimées dans le référentiel $R_1$, ces points ayant été soit acquis au préalable sur la structure non homogène SNH par exemple au moyen de la pointe de touche 30 puis soumis au transfert de référentiel, soit sélectionnnés directement sur la représentation au moyen de l'interface graphique de type "souris".

Le premier sous-module 3010 est suivi d'un deuxième sous-module noté 3011 permettant la création d'une matrice V tridimensionnelle orthogonale et unitaire, caractéristique d'une base orthonormée directe $\vec{i''}$, $\vec{j''}$, $\vec{k''}$, les vecteurs unitaires $\vec{i''}$, $\vec{j''}$, $\vec{k''}$ étant déterminés par les relations :

$$\vec{k''} = \vec{AB}/|\vec{AB}| ;$$
$$\vec{i''}.\vec{k''} = O \; ; \quad \vec{i''}.\vec{z''} = O \; ; \quad |\vec{i''}| = 1 \; ;$$
$$\vec{j''} = \vec{k''} \wedge \vec{i''}$$

où "$\wedge$" représente le produit vectoriel et "." symbolise le produit scalaire.

Le sous-module 3011 est suivi d'une routine 3012 permettant d'assurer pour tous les points des entités (structures d'intérêt) de la base de données tridimensionnelles de coordonnées XW, YW, ZW dans $R_1$ une conversion dans la base orthonormée ($\vec{i''}$, $\vec{j''}$, $\vec{k''}$) par la relation :

$$\{XV, YV, ZV\} = V * \{XW, YW, ZW\}$$

La sous-routine 3013 est alors suivie d'une sous-routine 3014 d'affichage du plan $\vec{i''}$, $\vec{j''}$, les sous-routines 3013 et 3014 étant rappelées pour tous les points, ainsi que symbolisé par la flèche de retour au bloc 3012 sur la figure 9a.

Lorsque l'ensemble des points a été traité, un module de sortie 3015 permet le retour à un module général, lequel sera décrit ultérieurement dans la description. On comprend que ce module permet de reconstruire des images bidimensionnelles dans des plans perpendiculaires à la direction définie par A et B.

De la même manière, le deuxième module 302 (figure 9b) de visualisation de la représentation selon un point de vue donné par un angle de site et un angle d'azimut comporte un premier sous-module 3020 d'acquisition des deux angles dans le repère représentation.

Le choix des angles de site et d'azimut peut être effectué par choix dans une base de données prédéfinies ou par déplacement de curseurs logiciels associés à chaque vue ou encore par modification relativement à une direction courante, telle que la direction modélisée d'intervention. Le sous-module 3020 est lui-même suivi d'un deuxième sous-module 3021 de création d'une matrice W tridimensionnelle orthogonale unitaire, caractéristique d'une base orthonormée directe de vecteurs unitaires $\vec{i'''}$, $\vec{j'''}$, $\vec{k'''}$. Ils sont définis par les relations :

$$\vec{k'''}.\vec{z'''} = \sin(\text{azimut}) \; ;$$
$$\vec{i'''}.\vec{k'''} = O \; ; \; \vec{j'''}.\vec{z'''} = O \; ; \; \vec{i'''}.\vec{y'''} = \cos(\text{site}) \; ;$$
$$\vec{i'''}.\vec{x'''} = \sin(\text{site}) \; ;$$
$$\vec{j'''} = \vec{k'''} \wedge \vec{i'''}$$

Une routine 3022 est ensuite appelée pour tous les points des entités de la base de données tridimensionnelles de coordonnées XW, YW, ZW, et permet d'appeler une première sous-routine 3023 permettant le calcul des coordonnées du point considéré dans la base orthonormée directe $\vec{i'''}$, $\vec{j'''}$, $\vec{k'''}$ par la transformation :

$$\{XV, YV, ZV\} = V * \{XW, YW, ZW\}$$

La sous-routine 3023 est elle-même suivie d'une sous-routine 3024 d'affichage du plan $\vec{i'''}$, $\vec{j'''}$, les deux sous-routines 3023 et 3024 étant alors appelées pour chaque point, ainsi que symbolisé par le retour par la flèche au bloc 3022 d'appel de la routine précitée. Lorsque tous les points ont été traités, un sous-module de sortie 3025 permet le retour au menu général.

Bien entendu, l'ensemble des programmes, sous-programmes, modules, sous-modules et routines précédemment décrits sont gérés par un programme général de type "menu" afin de permettre la conduite interactive du système par dialogue sur écran avec le praticien intervenant, par pages-écrans spécifiques.

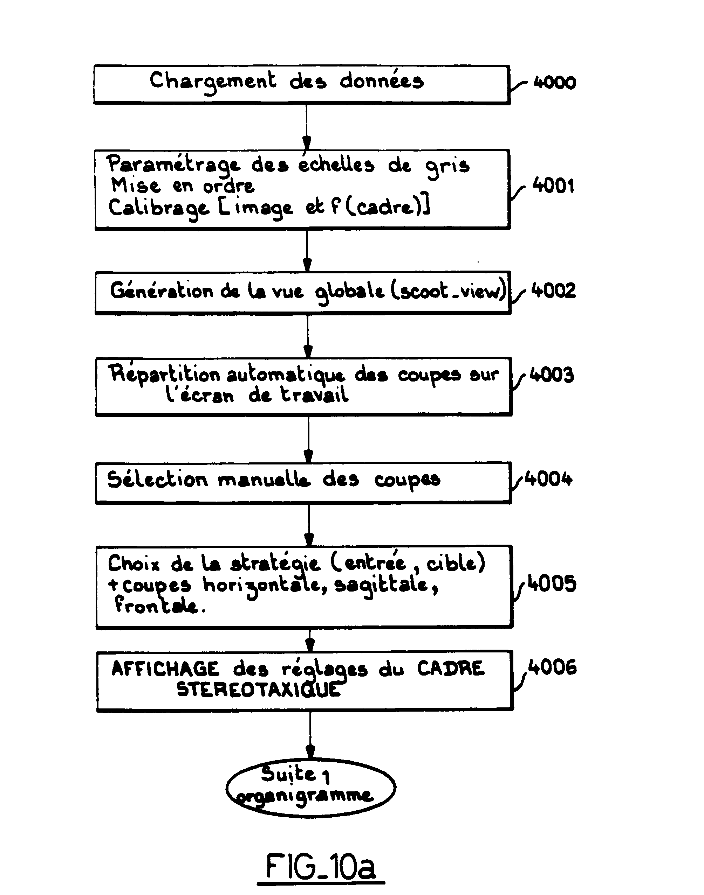

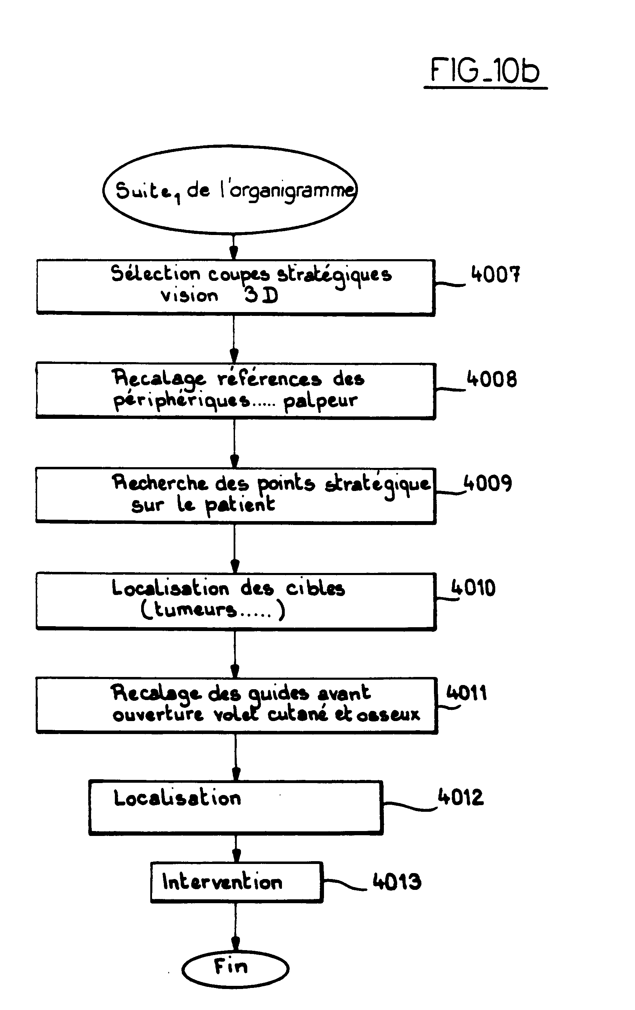

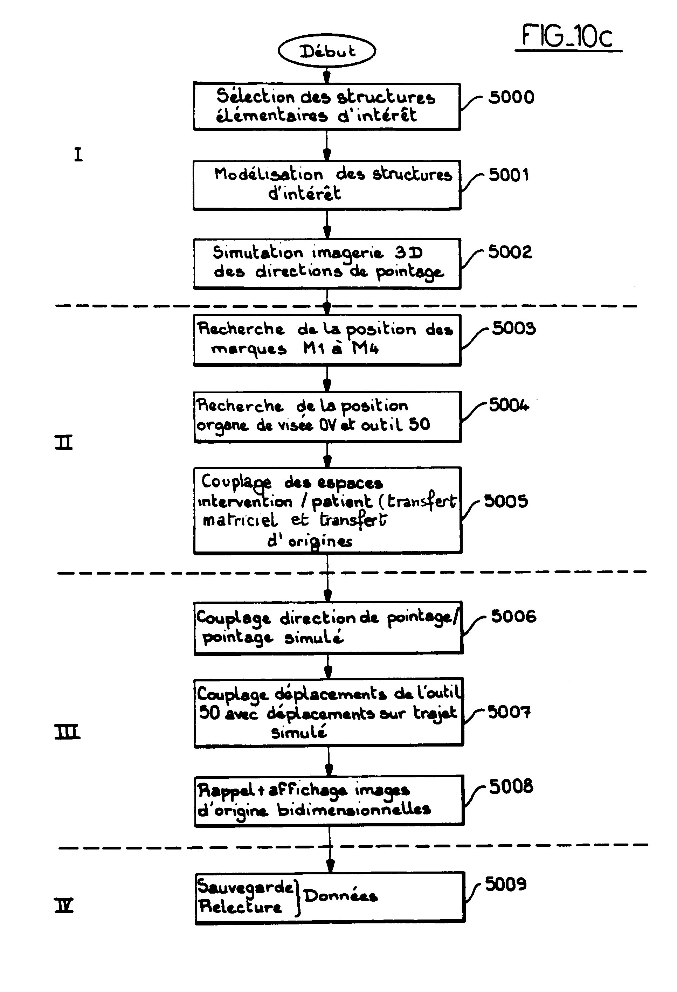

Une description plus spécifique d'un organigramme général illustrant ce programme général sera maintenant donnée en liaison avec les figures 10a et 10b.

Ainsi, sur la figure 10a, on a représenté successivement une page écran 4000 relative au chargement des données à partir du fichier numérisé 10, suivie d'une page écran 4001 permettant d'assurer le paramétrage des échelles de gris de l'affichage sur les moyens d'affichage dynamique 1 et de calibrage de l'image, par exemple.

La page écran 4001 est suivie d'une page écran 4002 permettant d'effectuer la génération d'une vue globale puis une étape ou page écran 4003 permet d'effectuer une répartition automatique des coupes sur l'écran de la station de travail.

Une page écran 4004 permet d'effectuer une sélection manuelle des coupes puis une page écran 4005 permet d'effectuer le choix de la stratégie (recherche des points d'entrée et des directions d'intervention possibles, première localisation de la cible (tumeur...) à traiter...), ainsi que défini précédemment, et de choisir la position et la répartition des coupes horizontale, sagittale et frontale.

Une page écran 4006 permet également d'effectuer un affichage des réglages d'un éventuel cadre stéréotaxique.

On rappellera que la structure de référence SR remplace avantageusement les cadres stéréotaxiques utilisés antérieurement pour effectuer le repérage de position à l'intérieur du crâne du patient.

Peuvent en outre être prévus une page écran 4007 de sélection des coupes stratégiques en vision tridimensionnelle au choix du praticien puis en 4008 le recalage des références des périphériques (outil, organes de visée, etc.) à l'aide du palpeur 30.

Une page écran 4009 est également prévue pour effectuer la recherche des points de base sur le patient à l'aide dudit palpeur, à la suite de quoi les étapes de construction des outils de transfert de référentiel et de transfert de référentiel à proprement-parler sont effectués, de façon préférentiellement transparente pour l'utilisateur.

Une autre page écran 4010 est alors prévue, afin d'effectuer la localisation de la cible sur la représentation (par exemple une tumeur à observer ou traiter dans le cas d'une intervention de neurochirurgie), pour déterminer ensuite un trajet simulé d'intervention.

Puis une nouvelle page écran 4011 permet d'effectuer le réglage des guides de l'outil à partir de ce trajet simulé avant ouverture des volets cutanés et osseux sur le crâne du patient.

Puis une nouvelle étape 4012 de localisation permet de vérifier que la position des guides correspond bien au trajet d'intervention simulé.

La page écran 4012 est suivie d'une page écran dite d'intervention, l'intervention étant effectuée conformément à l'étape 1110 de la figure 5b.

Une description plus détaillée du dialogue interactif entre le praticien et le système au cours d'une intervention chirurgicale et en particulier de neurochirurgie, va suivre en référence à la figure 10c et à l'ensemble de la description qui précède.

Les étapes de la figure 10c sont également intégrées au programme général précédemment mentionné; sont successivement déroulées une première phase I (préparation de l'intervention), puis une deuxième phase II, (préalablement à l'intervention proprement-dite, le patient est placé en condition d'intervention, la structure de référence SR étant liée au deuxième référentiel $R_2$), puis une troisième phase III (intervention) et enfin une phase IV postérieure à l'intervention.

En vue de préparer l'intervention, le système demande au praticien (étape 5000) d'effectuer une sélection des structure élémentaires d'intérêt (par exemple os du crâne, ventricules, zones vasculaires, la tumeur à explorer ou à traiter, ainsi que les images des marques constituant dans le premier référentiel la représentation RSR).

La sélection des structures élémentaires d'intérêt est effectuée sur l'affichage des images de tomographie par exemple, appelées à partir du fichier numérisé 10.

Le système effectue ensuite, à l'étape 5001, une modélisation des structures d'intérêt, ainsi que décrit précédemment. Puis, la structure non homogène ayant été ainsi constituée en un modèle tridimensionnel RSNH affiché sur l'écran, le praticien intervenant est alors amené à effectuer une simulation en imagerie tridimensionnelle, à l'étape 5002, en vue de définir le trajet d'intervention de l'outil 50.

Au cours de la phase II, le patient étant placé en condition d'intervention et sa tête et la structure de référence SR étant liées au deuxième référentiel $R_2$, le praticien effectue à l'étape 5003 une recherche de la position des marques M1 à M4 constituant des points de base de la structure de référence dans le deuxième référentiel $R_2$, puis au cours d'une étape 5004, effectue une recherche de la position des systèmes de visée, organe de visualisation OV, ou des outils et instruments d'intervention 50, toujours dans le deuxième référentiel $R_2$, afin le cas échéant de recaler ces appareils par rapport à $R_2$.

Puis le système effectue la validation des espaces intervention/patient et représentation en imagerie tridimensionnelle pour déterminer ensuite l'origine commune d'intervention ORI. En d'autres termes, le transfert matriciel de référentiel décrit plus haut est complété par les translations d'origines nécessaires (origines O1 et O2 recalées sur ORI).

Cette opération est effectuée comme décrit précédemment.

La phase III correspond à l'intervention, au cours de laquelle le système effectue à l'étape 5006 un couplage permanent et en temps réel entre la direction de pointage de l'organe actif 50, et ou de la direction de pointage de l'organe de visée OV (et le cas échéant du faisceau Laser), avec la direction de pointage (d'observation) simulée en imagerie tridimensionnelle sur le moyen d'affichage 1, et réciproquement.

A l'étape suivante 5007, on effectue le couplage des déplacements et mouvements de l'instrument d'intervention avec leurs déplacements simulés en imagerie tridimensionnelle, avec conduite de l'intervention en automatique ou en manuel.

Ainsi que noté en 5008, on peut fournir au praticien un affichage permanent des images d'origine en coupe bidimensionnelle dans des plans déterminés par rapport à l'origine ORI et à la direction d'intervention. Un tel affichage permet au praticien de suivre à tout moment le déroulement de l'intervention en temps réel et de s'assurer de la conformité de l'exécution de l'intervention par rapport à l'intervention silmulée. Dans la phase IV se déroulant après l'intervention, le système effectue une sauvegarde des données acquises au cours de l'intervention, cette sauvegarde permettant d'effectuer ensuite une comparaison en temps réel ou en différé en cas d'interventions successives sur le même patient.

Par ailleurs, les données sauvegardées permettent d'effectuer une relecture des opérations réalisées avec possibilité de détailler et compléter les zones traversées par l'organe actif 50.

On a ainsi décrit un système interactif d'intervention locale particulièrement performant.

Ainsi, le système, objet de la présente invention, permet-il de représenter un modèle ne comportant que les structures essentielles de la structure non homogène, ce qui facilite le travail de préparation et de contrôle de l'intervention par le praticien.

De plus, le système, grâce aux alorithmes utilisés et en particulier en minimisant la distorsion entre les points de base réels et leurs images dans les coupes 2D ou les atlas, permet d'établir un couplage bidirectionnel entre l'univers réel et l'univers informatique par lequel les erreurs de transfert sont minimisées, permettant une exploitation concrète des données d'imagerie pour asservir l'outil d'intervention.

En résumé, le système permet une utilisation médicale ineractive non seulement pour créer un modèle tridimensionnel de la structure non homogène mais également pour permettre un repérage en temps réel par raport aux structures internes et guider le praticien dans la phase d'intervention.

Plus généralement, l'invention permet d'aboutir à un système cohérent entre :

EP 0 494 943 B1

- les données d'imagerie bidimensionnelle (coupes scanner, atlas,...);
- la base de données tridimensionnelles;
- les données fournies par les moyens de repérage 3 dans le référentiel $R_2$;
- les données de coordonnées des systèmes de visée et outils d'intervention;
- l'univers réel du patient sur la table d'opération.

En conséquence, les possibilités offertes par le système sont, de façon non limitative, les suivantes:
- on peut représenter les outils et de leur position sur l'écran;
- la position d'un point sur l'écran peut être matérialisée sur le patient par exemple à l'aide du dispositif d'émission laser EL;
- l'orientation et le trajet d'un outil tel qu'une aiguille peut être représentée sur l'écran et matérialisée sur le patient de façon optique (émission laser) ou mécanique (positionnement du bras-guide dans lequel l'outil est guidé en translation);
- une image du patient, donnée par exemple par un système de prise de vues le cas échéant en relief, peut être superposée à la représentation tridimensionnelle modélisée sur l'écran; on peut ainsi visualiser toute modification des parties molles externes du patient par rapport à la saisie au scanner;
- le champ de visée du chirurgien donné par un organe de visée (tel que microscope chirurgical) pouvant être référencé par rapport à $R_2$, la direction de visualisation du modèle à l'écran peut être rendue identique à la vision réelle par l'organe de visée;
- enfin, les images tridimensionnelles, normalement affichées sur l'écran dans la description qui précède, peuvent en variante être injectées dans le microscope du praticien de manière à obtenir la superposition entre l'image réelle et la représentation du modèle.

**Revendications**

1. Système interactif d'intervention locale à l'intérieur d'une zone d'une structure non homogène (SNH) à laquelle est liée une structure de référence comportant une pluralité de points de base, du type comprenant :
   - des moyens d'affichage dynamique en imagerie tridimensionnelle d'une représentation de la structure non homogène et d'une structure de référence liée à la structure non homogène, incluant des images des points de base,
   - des moyens pour délivrer les coordonnées des images des points de base dans le premier référentiel ($R_1$),
   - des moyens (2) de mise en position liée, par rapport à un deuxième référentiel ($R_2$), de la structure non homogène et de la structure de référence,
   - des moyens (3) de repérage pour délivrer les coordonnées des points de base dans le deuxième référentiel,
   - des moyens d'intervention (5) comportant un organe actif dont la position est déterminée par rapport au deuxième référentiel,
   - des moyens (4) d'élaboration de jeux d'outils de transfert de référentiel (M, N) du premier référentiel vers le deuxième référentiel et réciproquement, à partir des coordonnées des images des points de base dans le premier référentiel et des coordonnées des points de base dans le deuxième référentiel,
   - des moyens pour définir par rapport au premier référentiel une origine d'intervention et une direction d'intervention simulées (ORI,$\Delta$), et
   - des moyens de transfert de référentiel (11) utilisant les dits outils de transfert de référentiel pour établir un couplage bi-directionnel entre l'origine d'intervention et la direction d'intervention simulées et la position de l'organe actif,

   caractérisé en ce que les moyens d'élaboration de jeux d'outils de transfert de référentiel sont aptes à élaborer au moins deux jeux d'outils à partir d'au moins deux triplets différents de points de base et d'images des points de base , en ce que le système comprend en outre des moyens (4) d'optimisation des outils de transfert de référentiel (M, N) aptes à valider un jeu d'outils de transfert de référentiel unique basé sur un triplet de points de base et un triplet d'images de ces points de base tels que les écarts entre les coordonnées d'au moins un autre point de base dans le deuxième référentiel et les coordonnées d'au moins un point image dudit autre point de base dans le premier référentiel soient réduits à un minimum.

2. système selon la revendication 1, caractérisé en ce que les moyens d'élaboration et d'optimisation des outils de transfert de référentiel comprennent :

    - un moyen (1002) pour créer des matrices (M) de transfert entre le premier référentiel et un premier référentiel intermédiaire basé sur les positions des images de trois points de base de la structure de référence,

    - un moyen (1004) pour créer des matrices (N) de transfert entre le deuxième référentiel et un deuxième référentiel intermédiaire basé sur les positions desdits trois points de base de la structure de référence, et

    - un moyen de validation (1005) de matrices (M,N) basées sur trois points de base et leurs images et tels que les écarts entre l'expression d'au moins un point de base additionnel dans le deuxième référentiel intermédiaire et l'expression d'au moins un point image de point de base additionnel dans le premier référentiel intermédiaire soient réduits à un minimum.

**3.** Système selon la revendication 1 ou 2, caractérisé en ce que les moyens d'élaboration et d'optimisation des outils de transfert de référentiel créent en association avec les outils de transfert de référentiel des outils d'affichage avec prise en compte d'une incertitude résiduelle, basés sur lesdits écarts réduits au minimum, lesdits outils d'affichage étant utilisables pour afficher dans la représentation au moins certains contours en tenant compte desdites incertitudes.

**4.** Système selon l'une des revendications 1 à 3, caractérisé en ce que lesdits moyens (1) d'affichage dynamique en imagerie tridimensionnelle comportent :

    - un fichier (10) de données numérisées d'images bidimensionnelles constituées par des coupes tomographiques successives non invasives et/ou des atlas de ladite structure non homogène (SNH),

    - des premiers moyens de calcul (110) et de reconstitution de ladite pluralité d'images tridimensionnelles à partir des données d'images bidimensionnelles,

    - un écran d'affichage haute résolution (12).

**5.** Système selon l'une des revendications précédentes, caractérisé en ce que les moyens (3) de repérage par rapport au deuxième référentiel ($R_2$) comprennent un palpeur tridimensionnel muni d'une pointe de touche (30) et délivrant les coordonnées (X2, Y2, Z2) de ladite pointe de touche dans ledit deuxième référentiel ($R_2$).

**6.** Système selon l'une des revendications précédentes, caractérisé en ce que les moyens de repérage par rapport au deuxième référentiel comprennent un jeu de capteurs optiques ou électromagnétiques.

**7.** Système selon l'une des revendications 1 à 6, caractérisé en ce qu'au moins une partie des points de base de la structure de référence (SR) consistent en une pluralité de marques (M1 à Mi) positionnées sur une surface latérale de la structure non homogène.

**8.** Système selon la revendication 7, caractérisé en ce que lesdites marques sont au nombre de quatre et sont distribuées sur ladite surface latérale de façon à définir un tétraèdre sensiblement symétrique.

**9.** Système selon la revendication 1, caractérisé en ce que lesdits moyens d'intervention sont constitués par :

    - un bras guide (51) susceptible d'assurer l'intervention dans la zone de la structure non homogène, ledit bras étant repéré en position instantanée par rapport audit deuxième référentiel ($R_2$), et

    - un organe actif d'intervention (50) dont la position est repérée par rapport audit deuxième référentiel.

**10.** Système selon la revendication 9, caractérisé en ce que, dans le cas d'une intervention chirurgicale intracranienne sur le cerveau, ledit organe actif d'intervention (50) est amovible et choisi dans le groupe comprenant :

    - les outils de trépanation.

    - les aiguilles et implants,

    - les têtes d'émission laser ou de radioéléments,

    - les systèmes de visée et de vision.

**11.** Système selon l'une des revendications 9 et 10, caractérisé en ce que ledit bras (51) est asservi en position par rapport au deuxième référentiel, l'organe actif d'intervention étant guidé en translation sur ledit bras par couplage via les outils de transfert de référentiel avec l'origine d'intervention (ORI) et la direction d'intervention (Δ) simulées.

12. Système selon l'une des revendications 10 à 12, caractérisé en ce qu'il comprend un programme général permettant, dans le cas d'une intervention chirurgicale, d'effectuer les opérations suivantes :
   - en vue de préparer l'intervention, à partir de ladite représentation en imagerie tridimensionnelle :
      . sélection par le praticien des structures élémentaires d'intérêt de la structure non homogène telles que os, ventricules, zones vasculaires, tumeur à observer ou traiter, et des images des points de base,
      . modélisation des structures élémentaires d'intérêt pour définir la représentation de la structure non homogène et de la structure de référence,
      . simulation en imagerie tridimensionnelle des directions de pointage, trajectoires et déplacements des outils et instruments pour atteindre et assurer l'intervention sur l'une des structures élémentaires d'intérêt déterminée,
   - préalablement à l'intervention, le patient étant placé en condition d'intervention et la structure de référence (SR) étant liée audit deuxième référentiel ($R_2$):
      . recherche, à l'aide des moyens de repérage (3), de la position des points de base (M1 à M4) de la structure de référence (SR) dans ledit deuxième référentiel ($R_2$),
      . recherche, à l'aide des moyens de repérage (3), de la position des systèmes de visée (OV) ou outils et de l'organe actif (50) dans ledit deuxième référentiel ($R_2$),
      . élaboration des outils de transfert de référentiel (M, N),
   - pendant l'intervention, à l'aide des moyens (11) de transfert de référentiel:
      . couplage de la direction de pointage (Δ) de l'instrument d'intervention avec la direction de pointage simulée en imagerie tridimensionnelle sur les moyens d'affichage (1),
      . couplage des déplacements et mouvements de l'instrument d'intervention avec leurs déplacements simulés en imagerie tridimensionnelle,
      . création et affichage d'images bidimensionnelles dans des plans déterminés par rapport à l'origine et à la direction d'intervention simulées,
   - après l'intervention:
      . sauvegarde pour comparaison en temps réel ou en différé en cas d'interventions successives sur le même patient,
      . relecture des opérations réalisées avec possibilité de détailler et compléter les zones traversées par l'instrument d'intervention.

13. Système selon la revendication 4, caractérisé en ce que lesdits premiers moyens de calcul (110) et de reconstitution de ladite pluralité d'images tridimensionnelles à partir de données d'images bidimensionnelles comportent un programme constitué par un logiciel de type CAO.

14. Système selon la revendication 2, caractérisé en ce que les moyens de transfert de référentiel comprennent:
   - un premier sous-module (201) de transfert de coordonnées représentation/structure non homogène, et
   - un deuxième sous-module (202) de transfert de coordonnées structure non homogène/représentation.

15. Système selon la revendication 14, caractérisé en ce que ledit premier sous-module (201) de transfert comprend :
   - une procédure (2010) d'acquisition des coordonnées (XM, YM, ZM), exprimées dans le premier référentiel ($R_1$), du point de la représentation à transférer, par sélection sur la représentation,
   - une procédure (2011) de calcul des coordonnées (XP, YP, ZP) correspondantes, exprimées dans le deuxième référentiel ($R_2$), sur la structure non homogène par la transformation :

$$\{YP, YP, ZP\} = M * N^{-1} * \{XM, YM, ZM\}$$

où $M * N^{-1}$ représente le produit de la matrice M et de la matrice N inverse, et
   - une procédure (2012) de traitement à l'aide des coordonnées (YP, YP, ZP) par le système d'intervention pour afficher le point correspondant sur la surface de la structure non homogène (SNH) ou pour assurer l'intervention.

EP 0 494 943 B1

**16.** Système selon la revendication 14 ou 15, caractérisé en ce que ledit deuxième sous-module (202) de transfert comprend :
- une procédure (2020) d'acquisition des coordonnées (XP, YP, ZP), exprimées dans le deuxième référentiel ($R_2$), du point de la structure non homogène à transférer,
- une procédure (2021) de calcul des coordonnées (XP, YP, ZP) correspondantes, exprimées dans le premier référentiel ($R_1$), sur la représentation par la transformation :

$$\{YM, YM, ZM\} = N * M^{-1} * \{XP, YP, ZP\}$$

où $N * M^{-1}$ représente le produit de la matrice N et de la matrice M inverse, et
- une procédure (2022) d'affichage à l'aide des des coordonnées (YM, YM, ZM) dans la représentation par les moyens (1) d'affichage dynamique.

**17.** Système selon la revendication 1, caractérisé en ce que les moyens de transfert de référentiel sont utilisés pour établir un couplage entre la direction de visualisation de la représentation de la structure non homogène (RSNH) sur les moyens d'affichage et la direction d'observation de la structure non homogène et de la structure de référence par l'organe actif.

**18.** Système selon la revendication 17, caractérisé en ce qu'il est prévu :
- un premier module (301) de visualisation de la représentation dans une direction donnée par deux points,
- un deuxième module (302) de visualisation de la représentation dans une direction donnée par un angle de site et un angle d'azimut.

## Claims

**1.** A local operation interactive system inside an area of a non-homogeneous structure (NHS) to which is linked a reference structure including a plurality of base points, of a type comprising :
- three-dimensional imaging dynamic display means for displaying a representation of said non-homogeneous structure and of a reference structure linked to said non-homogeneous structure, including images of said base points,
- means for delivering the coordinates of images of said base points in a first referential ($R_1$),
- means (2) for putting in a linked position, with reference to a second referential ($R_2$), both non-homogeneous and reference structures,
- locating means for delivering the coordinates of base points into said second referential,
- operation means (5) including an active member, the position of which is set with reference to said second referential,
- means (4) for elaborating referential transfer tool sets (M, N) from said first referential to said second referential and conversely, through coordinates of base point images in said first referential and coordinates of base points in said second referential,
- means for defining with reference to said first referential, both simulated operation origin and operation direction (ORI, $\Delta$) and
- referential transfer means (11) using said referential transfer tools so as to establish a bidirectional coupling between said simulated operation origin and operation direction and said active member position,

characterized in that said means for elaborating referential transfer tool sets are suitable to elaborate at least two tool sets from at least two different triplets of base points and base points images, in that said system further includes means (4) for optimizing referential transfer means (M, N) suitable to validate an unique referential transfer tool set based on a triplet of base points and a triplet of images of those base points such that deviations between coordinates of at least another base point in said second referential and coordinates of at least an image point of said other base point in said first referential are reduced to a minimum.

**2.** The system as recited in claim 1, characterized in that said means for elaborating and optimizing referential transfer tools include :
- a means (1002) for creating matrixes (M) for a transfer action between said first referential and a first intermediate referential based upon the image positions of three base points of said reference structure,

18

- a means (1004) for creating matrixes (N) for a transfer action between said second referential and a second intermediate referential based upon the positions of said three base points of said reference structure, and
- a means (1005) for validating matrixes (M, N) based upon three base points and images thereof and in such a way that deviations between the expression of at least on additional base point in said second intermediate referential and the expression of at least an image point of an additional base point in said first intermediate referential are reduced to a minimum.

3. The system as recited in claim 1 or 2, characterized in that said means for elaborating and optimizing said referential transfer tools create together with said referential transfer tools, display tools taking into account a residual uncertainty, based upon said deviations reduced to a minimum, said display tools being available to display in the representation at least some contours taking into account said uncertainties.

4. The system as recited in one of claims 1 to 3, characterized in that said means (1) for three-dimensional imaging dynamic display include :
- a file (10) of digitalized data of two-dimensional images comprising successive non-invasive tomographic sections and/or atlases of said non-homogeneous structure (SNH),
- first means (110) for calculating and for reconstituting said plurality of said three-dimensional images from said two-dimensional image data,
- a high resolution display screen (12).

5. The system as recited in any one of the preceding claims, characterized in that said locating means (3) for carrying out a locating action with reference to said second referential ($R_2$) include a three-dimensional feeler provided with a touch style (30), and delivering coordinates (X2, Y2, Z2) of said touch style in said second referential ($R_2$).

6. The system as recited in one of the preceding claims, characterized in that said locating means with reference to said second referential include a set of optical or electromagnetic sensors.

7. The system as recited in one of claims 1-6, characterized in that at least a part of said base points of said reference structure (SR) comprises a plurality of marks (Ml to Mi) positioned on a lateral surface of said non-homogeneous structure.

8. The system as recited in claim 7, characterized in that said marks are four in number and are distributed on said lateral surface so as to define a substantially symetrical tetrahedral.

9. The system as recited in claim 1, characterized in that said operation means include :
- a guiding arm (51) capable of insuring an operation in the non-homogeneous structure area, said arm being located in an instantaneous position with reference to said second referential ($R_2$), and
- an operation active member (50) the position of which is located with reference to said second referential.

10. The system as recited in claim 9, characterized in that, should an intracranial chirurgical operation be carried out, said operation active member (50) is removable and choosen in the group including :
- trepanning tools,
- needles and implants,
- laser or radio-element emitting heads,
- sight and vision systems.

11. The system as recited in one of claims 9 and 10, characterized in that said arm (51) is positionaly controlled relative to said second referential, said operation active member being translation guided on said arm by a coupling action via said referential transfer tools with said simulated operation origin (OOR) and operation direction (Δ).

12. The system as recited in anyone of claims 10-12, characterized in that it includes a general program allowing, should a chirurgical operation occur, the following actions to be carried out :
- in view of preparing said operation, from said three-dimensional imaging representation :

. selection by practitioner of elementary structures of interest of said non-homogeneous structure such as bones, ventricules, vascular areas, tumor to be observed or treated, and of the images of said base points,

. modelization of elementary structures of interest for defining the representation of both non-homogeneous structure and reference structure,

. simulation in three-dimensional imaging of the pointing directions, paths and movements of tools and instruments in order to reach and insure the operation on a determined one of said elementary structures of interest,

- prior to the operation, with the patient being placed in operation condition and with the reference structure (RS) being linked to said second referential ($R_2$)

. search, with the help of said locating means (3), of said base points (M1-M4) position of said reference structure (RS) in said second referential ($R_2$),

. search, with the help of said locating means (3), of the position of said sight systems (ov) or tools and of the active member (50) in said second referential ($R_2$),

. elaboration of referential transfer tools (M, N),

- during the operation, with the help of said referential transfer means (11),

. coupling the pointing direction (Δ) of the operation instrument with the simulated pointing direction in three-dimensional imaging on said display means (1),

. coupling the displacements and movements of the operation instrument with their simulated displacements in three-dimensional imaging,

. creation and display of two-dimensional images in planes determined with reference to the simulated origin and direction of the operation,

- after the operation :

. safeguard for real time or delayed comparison in case of successive operations on the same patient,

. re-reading of the realized operations with the possibility of detailing and completing the areas traversed by the operation instrument.

13. The system as recited in claim 4, characterized in that said first means (110) for calculating and reconstituting said plurality of three-dimensional images from two-dimensional image data include a program incorporating CAD type software.

14. The system as recited in claim 2, characterized in that said referential transfer means include :

- a first sub-module (201) for transferring representation/non-homogeneous structure coordinates, and
- a second sub-module (202) for transferring non-homogeneous structure/representation coordinates.

15. The system as recited in claim 14, characterized in that said first transfer sub-module (201) includes :

a procedure (2010) for acquiring coordinates (XM, YM, ZM), expressed in said first referential ($R_1$) of the point of the representation to be transferred, by selecting on said representation,

a procedure (2011) for calculating corresponding coordinates (XP, YP, ZP), expressed in the second referential ($R_2$), on said non-homogeneous structure by the following transform :

$$\{YP, YP, ZP\} = M * N^{-1} * \{XM, YM\ ZM\}$$

wherein $M * N^{-1}$ is the product of matrix M by inverse matrix N, and

- a treatment procedure (2012) by means of coordinates (XP, YP, ZP) by said operation system in order to display the corresponding point on the surface of said non-homogeneous structure (NHS) or to ensure the operation.

16. The system as recited in claim 14 or 15, characterized in that said second transfer sub-module (202) includes :

- a procedure (2020) for acquiring coordinates (XP, YP, ZP), expressed in said second referential ($R_2$), of the non-homogeneous structure point to be transferred,
- a procedure (2021) for calculating corresponding coordinates (XP, YP, ZP), expressed in the first referential (R1), on said representation by the following transform :

$$\{YM, YM, ZM\} = N * N^{-1} * \{XP, YP, ZP\}$$

wherein $N * N^{-1}$ shows the product of matrix N and inverse matrix M and
- a display procedure (2022) with the help of coordinates (YM, YM, ZM) in the representation by said dynamic display means (1).

17. The system as recited in claim 1, characterized in that said referential transfer means are used to establish a coupling between the direction for visualizing the non-homogeneous structure representation (NHSR) on said display means and the direction for observing said non-homogeneous structure and reference structure by said active member.

18. The system as recited in claim 17, wherein :
- a first module (301) for visualizing said representation in a direction given by two points,
- a second module (302) for visualizing said representation in a direction given by an angle of site and an azimuth angle, are provided.

**Patentansprüche**

1. Interaktives System für einen lokalen Eingriff im Inneren einer Zone einer nicht homogenen Struktur (NHS), mit der eine Referenzstruktur verbunden ist, die eine Mehrzahl von Basispunkten aufweist, von der Art, umfassend:
- Mittel zur dynamischen Anzeige einer Abbildung der nicht homogenen Struktur und einer mit der nicht homogenen Struktur verbundenen Referenzstruktur, welche die Bilder der Basispunkte einschließt, in Form dreidimensionaler Bilder,
- Mittel, welche die Koordinaten der Bilder der Basispunkte in dem ersten Bezugssystem ($R_1$) liefern,
- Mittel (2), um die nicht homogene Struktur und die Referenzstruktur in bezug auf ein zweites Bezugssystem ($R_2$) in eine feste Position zu bringen,
- Ortungsmittel (3), welche die Koordinaten der Basispunkte in dem zweiten Bezugssystem liefern,
- Mittel für den Eingriff (5), welche ein aktives Organ umfassen, dessen Position in bezug auf das zweite Bezugssystem bestimmt wird,
- Mittel (4) zu Ausarbeitung von Instrumentarien zur Bezugssystemübertragung (M, N) des ersten Bezugssystems auf das zweite Bezugssystem und umgekehrt anhand der Koordinaten der Bilder der Basispunkte in dem ersten Bezugssystem und der Koordinaten der Basispunkte in dem zweiten Bezugssystem,
- Mittel, um einen simulierten Eingriffsnullpunkt und eine simulierte Eingriffsrichtung (ORI,Δ) in bezug auf das erste Bezugssystem zu definieren, und
- Mittel zur Bezugssystemübertragung (11), welche das genannte Instrumentarium zur Bezugssystemübertragung verwenden, um eine bidirektionale Kopplung zwischen dem simulierten Eingriffsnullpunkt und der simulierten Eingriffsrichtung und der Position des aktiven Organs herzustellen,
dadurch gekennzeichnet, daß die Mittel zur Ausarbeitung von Instrumentarien zur Bezugssystemübertragung dafür vorgesehen sind, mindestens zwei Instrumentarien anhand mindestens zweier verschiedener Tripletts von Basispunkten und Bildern der Basispunkte auszuarbeiten, daß das System außerdem Mittel (4) zur Optimierung des Instrumentariums zur Bezugssystemübertragung (M, N) umfaßt, dafür vorgesehen, ein einziges auf einem Triplett von Basispunkten und einem Triplett von Bildern dieser Basispunkte beruhendes Instrumentarium zur Bezugssystemübertragung gültig zu machen, das so beschaffen ist, daß die Abstände zwischen den Koordinaten mindestens eines weiteren Basispunkts in dem zweiten Bezugssystem und den Koordinaten mindestens eines Bildpunkts dieses weiteren Basispunkts in dem ersten Bezugssystem auf ein Minimum reduziert sind.

2. System nach Anspruch 1, dadurch gekennzeichnet, daß die Mittel zur Ausarbeitung und Optimierung des Instrumentariums zur Bezugssystemübertragung umfassen:
- ein Mittel (1002) zur Schaffung von Matrizes (M) zur Übertragung zwischen dem ersten Bezugssystem und einem ersten Zwischenbezugssystem auf der Basis der Positionen der Bilder von drei Basispunkten der Referenzstruktur,
- ein Mittel (1004) zur Schaffung von Matrizes (N) zur Übertragung zwischen dem zweiten Bezugssystem und einem zweiten Zwischenbezugssystem auf der Basis der Positionen der genannten drei Basispunkte der Referenzstruktur, und

- ein Mittel zur Validierung (1005) von Matrizes (M, N), die auf drei Basispunkten und ihren Bildern basieren und derart beschaffen sind, daß die Abstände zwischen dem Ausdruck mindestens eines zusätzlichen Basispunkts in dem zweiten Zwischenbezugssystem und dem Ausdruck mindestens eines Bildpunkts des zusätzlichen Basispunkts in dem ersten Zwischenbezugssystem auf ein Minimum reduziert sind.

3. System nach Anspruch 1 oder 2, dadurch gekennzeichnet, daß die Mittel zur Ausarbeitung und Optimierung des Instrumentariums zur Bezugssystemübertragung unter Einbeziehung des Instrumentariums zur Bezugssystemübertragung ein Instrumentarium zur Anzeige unter Berücksichtigung einer Restunsicherheit schaffen, das auf den auf ein Minimum reduzierten Abständen beruht, wobei dieses Anzeigeinstrumentarium verwendet werden kann, um in der Abbildung mindestens bestimmte Umrisse unter gleichzeitiger Berücksichtigung dieser Unsicherheiten anzuzeigen.

4. System nach einem der Ansprüche 1 bis 3, dadurch gekennzeichnet, daß die Mittel (1) zur dynamischen Anzeige in Form dreidimensionaler Bilder umfassen:

- eine Datei (10) von digitalisierten Daten zweidimensionaler Bilder, bestehend aus nicht invasiven aufeinanderfolgenden tomographischen Schnitten und/oder Atlanten der nicht homogenen Struktur (NHS),
- erste Mittel (110) zur Berechnung und Rekonstruktion einer Vielzahl dreidimensionaler Bilder anhand der Daten zweidimensionaler Bilder,
- einen Anzeigebildschirm mit hoher Auflösung (12).

5. System nach einem der vorhergehenden Ansprüche, dadurch gekennzeichnet, daß die Mittel (3) zur Ortung in bezug auf das zweite Bezugssystem ($R_2$) einen dreidimensionalen Taster umfassen, der mit einer Tastspitze (30) versehen ist und die Koordinaten (X2, Y2, Z2) dieser Tastspitze in dem zweiten Bezugssystem ($R_2$) liefert.

6. System nach einem der vorhergehenden Ansprüche, dadurch gekennzeichnet, daß die Mittel zur Ortung in bezug auf das zweite Bezugssystem einen Satz optischer oder elektromagnetischer Sensoren umfassen.

7. Mittel nach einem der Ansprüche 1 bis 6, dadurch gekennzeichnet, daß zumindest ein Teil der Basispunkte der Referenzstruktur (RS) aus einer Mehrzahl von Markierungen (M1 bis Mi) besteht, die auf einer Seitenfläche der nicht homogenen Struktur angebracht sind.

8. System nach Anspruch 7, dadurch gekennzeichnet, daß die Zahl der genannten Markierungen vier ist und die Markierungen auf der Seitenfläche so verteilt sind, daß sie einen im wesentlichen symmetrischen Tetraeder definieren.

9. System nach Anspruch 1, dadurch gekennzeichnet, daß die Mittel zum Eingriff gebildet werden von:

- einem Führungsarm (51), welcher den Eingriff in der Zone der nicht homogenen Struktur gewährleisten kann, wobei die jeweils augenblickliche Position dieses Arms in bezug auf das zweite Bezugssystem ($R_2$) ermittelt wird, und
- einem aktiven Eingriffsorgan (50), dessen Position in bezug auf das zweite Bezugssystem ermittelt wird.

10. System nach Anspruch 9, dadurch gekennzeichnet, daß im Falle eines intrakranialen chirurgischen Eingriffs am Hirn dieses aktive Eingriffsorgan (50) abnehmbar ist und aus der folgenden Gruppe gewählt wird:

- Trepanationsinstrumenten,
- Nadeln und Implantaten,
- Laseremissionsköpfen oder Köpfen zur Freisetzung von Radioisotopen,
- Peilungs- und Beobachtungssystemen.

11. System nach einem der Ansprüche 9 und 10, dadurch gekennzeichnet, daß der Arm (51) in seiner Position bezogen auf das zweite Bezugssystem gesteuert wird, wobei die Translationsbewegung des aktiven Eingriffsorgans auf dem Arm durch die über das Instrumentarium zur Bezugssystemübertra-

EP 0 494 943 B1

gung erfolgende Kopplung mit dem simulierten Eingriffsnullpunkt (ORI) und der simulierten Eingriffs-richtung ($\Delta$) gelenkt wird.

**12.** System nach einem der Ansprüche 10 bis 12, dadurch gekennzeichnet, daß es ein Grundprogramm umfaßt, das im Falle eines chirurgischen Eingriffs die Durchführung der folgenden Arbeitsschritte ermöglicht:
- in Hinsicht auf die Vorbereitung des Eingriffs anhand der Abbildung in Form dreidimensionaler Bilder:
  - die Auswahl der grundlegenden Strukturen der nicht homogenen Struktur, welche von Interesse sind, wie Knochen, Ventrikel, Gefäßzonen, der zu beobachtende oder zu behandelnde Tumor, und der Bilder der Basispunkte durch den Arzt,
  - die Modellierung der grundlegenden Strukturen von Interesse, um die Abbildung der nicht homogenen Struktur und der Referenzstruktur zu definieren,
  - die Simulation der Zielrichtungen, Bahnen und Verschiebungen der Geräte und Instrumente in dreidimensionalen Bildern, um den Eingriff an einer der festgelegten grundlegenden Strukturen von Interesse zu erreichen und zu gewährleisten,
- vor dem Eingriff, wobei der Patient auf den Eingriff vorbereitet wurde und die Referenzstruktur (RS) mit dem zweiten Bezugssystem ($R_2$) verbunden wurde:
  - die Bestimmung, mittels der Ortungsmittel (3), der Position der Basispunkte (M1 bis M4) der Referenzstruktur (RS) in dem zweiten Bezugssystem ($R_2$),
  - die Bestimmung, mittels der Ortungsmittel (3), der Position der Peilsysteme (PO) oder Geräte und des aktiven Organs (50) in dem zweiten Bezugssystem ($R_2$)
  - die Ausarbeitung des Instrumentariums zur Bezugssystemübertragung (M, N),
- während des Eingriffs, mit Hilfe der Mittel (11) zur Bezugssystemübertragung:
  - die Kopplung der Zielrichtung ($\Delta$) des Eingriffsinstruments mit der in dreidimensionalen Bildern auf den Anzeigemitteln (1) simulierten Zielrichtung,
  - die Kopplung der Verschiebungen und Bewegungen des Eingriffsinstruments mit ihren in dreidimensionalen Bildern simulierten Verschiebungen,
  - die Erzeugung und Anzeige zweidimensionaler Bilder in hinsichtlich des simulierten Nullpunkts und der simulierten Richtung des Eingriffs festgelegten Ebenen,
- nach dem Eingriff:
  - die Sicherung zum Vergleich in Echtzeit oder zu einem späteren Zeitpunkt im Falle von aufeinanderfolgenden Eingriffen am selben Patienten,
  - das erneute Lesen der durchgeführten Arbeitsschritte mit der Möglichkeit, die von dem Eingriffsinstrument durchwanderten Zonen zu präzisieren und zu vervollständigen.

**13.** System nach Anspruch 4, dadurch gekennzeichnet, daß die genannten ersten Mittel (110) zur Berechnung und Rekonstruktion der Vielzahl dreidimensionaler Bilder anhand von Daten zweidimensionaler Bilder ein Programm umfassen, das von einer Software des Typs CAO gebildet wird.

**14.** System nach Anspruch 2, dadurch gekennzeichnet, daß die Mittel zur Bezugssystemübertragung umfassen:
- einen ersten Submodul (201) zur Koordinatenübertragung Abbildung/nicht homogene Struktur, und
- einen zweiten Submodul (202) zur Koordinatenübertragung nicht homogene Struktur/Abbildung.

**15.** System nach Anspruch 14, dadurch gekennzeichnet, daß der erste Submodul (201) zur Übertragung umfaßt:
- ein Verfahren (2010) zur Erfassung der Koordinaten (XM, YM, ZM), ausgedrückt in dem ersten Bezugssystem ($R_1$), des zu übertragenden Punkts der Abbildung durch Auswahl auf der Abbildung,
- ein Verfahren (2011) zur Berechnung der entsprechenden Koordinaten (XP, YP, ZP), ausgedrückt in dem zweiten Bezugssystem ($R_2$), auf der nicht homogenen Struktur durch die Transformation:

$$\{XP, YP, ZP\} = M * N^{-1} * \{XM, YM, ZM\}$$

worin $M * N^{-1}$ das Produkt der Matrix M und der reziproken Matrix N darstellt, und

23

- ein Verfahren (2012) zur Behandlung durch das Eingriffssystem mit Hilfe der Koordinaten (XP, YP, ZP), um den entsprechenden Punkt auf der Oberfläche der nicht homogenen Struktur (NHS) anzuzeigen oder um den Eingriff vorzunehmen.

16. System nach Anspruch 14 oder 15, dadurch gekennzeichnet, daß der zweite Submodul (202) zur Übertragung umfaßt:
    - ein Verfahren (2020) zur Erfassung der Koordinaten (XP, YP, ZP), ausgedrückt in dem zweiten Bezugssystem ($R_2$), des zu übertragenden Punkts der nicht homogenen Struktur,
    - ein Verfahren (2021) zur Berechnung der entsprechenden Koordinaten (XM, YM, ZM), ausgedrückt in dem ersten Bezugssystem ($R_1$), auf der Abbildung durch die Transformation:

    $$\{XM, YM, ZM\} = N * M^{-1} * \{XP, YP, ZP\}$$

    worin $N * M^{-1}$ das Produkt der Matrix N und der reziproken Matrix M darstellt, und
    - ein Verfahren (2022) zur Anzeige mittels der Koordinaten (XM, YM, ZM) in der Darstellung durch die Mittel (1) zur dynamischen Anzeige.

17. Verfahren nach Anspruch 1, dadurch gekennzeichnet, daß die Mittel zur Bezugssystemübertragung verwendet werden, um eine Kopplung zwischen der Richtung der Sichtanzeige der Darstellung der nicht homogenen Struktur (DNHS) auf den Anzeigemitteln und der Richtung der Wahrnehmung der nicht homogenen Struktur und der Referenzstruktur durch das aktive Organ herzustellen.

18. System nach Anspruch 17, dadurch gekennzeichnet, daß vorgesehen ist:
    - ein erster Modul (301) zur Sichtanzeige der Darstellung in einer durch zwei Punkte vorgegebenen Richtung,
    - ein zweiter Modul (302) zur Sichtanzeige der Darstellung in einer durch einen Zielhöhenwinkel und einen Seitenwinkel vorgegebenen Richtung.

FIG.1

## FIG_2

## FIG_3

FIG_4

| Données images 2D | 100 |

| Sélection des structures d'intérêt | 101 |

| Génération de la base de données 3D | 102 |

| Appel logiciel de type CAO | 103 |

| Affichage écran haute résolution de : représentation de structure non homogène et de la structure de référence. | 104 |

| Simulation trajet intervention sur images 2D et 3D. | 105 |

Suite 1

FIG_5a

Suite 1

**107**
Repérage des points de base par rapport au deuxième référenciel R2

**106**
Repérage des images des points de base par rapport au premier référentiel $R_1$

**108**
Elaboration des Outils de transfert de référentiel et transfert d'origine

**109**
Test des 4 points

**1111**
Relevé de position 3D de l'outil d'intervention

**1110**
Intervention: asservissement de l'outil sur le trajet d'intervention simulée.

Fin

## FIG_5b

1001 — Aquisition des 3 points de référence sur la représentation A,B,C exprimés dans la base globale par sélection sur le modèle géométrique 3D

1002 — Création d'une matrice M 3x3 orthogonale, unitaire caractéristique d'une base orthonormée directe $(\vec{\imath},\vec{\jmath},\vec{k})$

1003 — Aquisition des 3 points de référence sur le PATIENT: D,E,F exprimés dans la base globale par digitalisation 3D

1004 — Création d'une matrice N 3x3 orthogonale, unitaire caractéristique d'une base orthonormée directe $(\vec{\imath'},\vec{\jmath'},\vec{k'})$

1005 — Validation: choix des matrices M , N après test sur point(s) de base additionnel(s).

FIG.6

30

Aquisition du point à transférer sur la représentation $(XM, YM, ZM)$ par sélection sur le modèle géométrique 3D — 2010

Calcul des coordonnées $(XP, YP, ZP)$ sur le patient par la transformation :

$$(XP, YP, ZP) = M \ast N^{-1} \ast (XM, YM, ZM)$$

— 2011

Traitement des coordonnées $(XP, YP, ZP)$ par le système d'intervention — 2012

201

FIG_7

Acquisition du point à transférer sur le patient (XP,YP,ZP) par digitalisation 3D

2020

Calcul des coordonnées (XM,YM,ZM) sur la représentation par la transformation :

$$(XM,YM,ZM) = N_a M^{-1}_a (XP,YP,ZP)$$

2021

Affichage du point (XM,YM,ZM) par le système de visualisation

2022

202

FIG_8

3010 — Aquisition des 2 points de la direction A,B en coordonnées représentation par digitalisation 3D sur le patient & transfert ou par sélection sur le modèle géométrique 3D

3011 — Création d'une matrice $\bigvee$ 3×3 orthogonale, unitaire caractéristique d'une base orthonormée directe $(\vec{i''}, \vec{j''}, \vec{k''})$

3012 — Pour tous les points de référence des entités de la base de données 3D $(Xw, Yw, Zw)$

3015 — SORTIE

3013 — Calcul des coordonnées du point dans la base $(i'', j'', k'')$ par la transformation

$$(Xv, Yv, Zv) = \bigvee * (Xw, Yw, Zw)$$

3014 — Affichage du plan $i'', j''$

301

## FIG.9a

Acquisition des deux angles de la direction: site et azimut dans le repère représentation  — 3020

Création d'une matrice $\boxed{W}$ 3 x 3 orthogonale, unitaire, caractéristique d'une base orthonormée directe $(\vec{i}''', \vec{j}''', \vec{k}''')$  — 3021

302

Pour tous les points de référence des entités de la base de données 3D $(Xw, Yw, Zw)$  — 3022

SORTIE — 3025

Calcul des coordonnées du point dans la base $(i''', j''', k''')$ par la transformation

$$(Xv, Yv, Zv) = \boxed{W} \bullet (Xw, Yw, Zw)$$

— 3023

Affichage du plan i,j — 3024

FIG_9b

Chargement des données — 4000

Paramétrage des échelles de gris
Mise en ordre
Calibrage [image et f(cadre)] — 4001

Génération de la vue globale (scoot.view) — 4002

Répartition automatique des coupes sur l'écran de travail — 4003

Sélection manuelle des coupes — 4004

Choix de la stratégie (entrée, cible)
+ coupes horizontale, sagittale, frontale. — 4005

AFFICHAGE des réglages du CADRE STEREOTAXIQUE — 4006

Suite 1 organigramme

FIG_10a

## FIG.10b

```
        ┌─────────────────────────────┐
        │  Suite, de l'organigramme   │
        └─────────────────────────────┘
                      │
                      ▼
   ┌──────────────────────────────────┐
   │  Sélection coupes stratégiques   │── 4007
   │        vision  3D                │
   └──────────────────────────────────┘
                      │
                      ▼
   ┌──────────────────────────────────┐
   │  Recalage références des         │── 4008
   │  périphériques..... palpeur      │
   └──────────────────────────────────┘
                      │
                      ▼
   ┌──────────────────────────────────┐
   │  Recherche des points stratégique│── 4009
   │        sur le patient            │
   └──────────────────────────────────┘
                      │
                      ▼
   ┌──────────────────────────────────┐
   │  Localisation des cibles         │── 4010
   │        (tumeurs..... )           │
   └──────────────────────────────────┘
                      │
                      ▼
   ┌──────────────────────────────────┐
   │  Recalage des guides avant       │── 4011
   │  ouverture volet cutané et osseux│
   └──────────────────────────────────┘
                      │
                      ▼
   ┌──────────────────────────────────┐
   │  Localisation                    │── 4012
   └──────────────────────────────────┘
                      │
                      ▼
        ┌─────────────────────┐
        │  Intervention       │── 4013
        └─────────────────────┘
                      │
                      ▼
              ┌──────────┐
              │   Fin    │
              └──────────┘
```

FIG.10c

Début

Sélection des structures élémentaires d'intérêt — 5000

I

Modélisation des structures d'intérêt — 5001

Simulation imagerie 3D des directions de pointage — 5002

Recherche de la position des marques M1 à M4 — 5003

Recherche de la position organe de visée OV et outil 50 — 5004

II

Couplage des espaces intervention / patient (transfert matriciel et transfert d'origines — 5005

Couplage direction de pointage / pointage simulé — 5006

Couplage déplacements de l'outil 50 avec déplacements sur trajet simulé — 5007

III

Rappel + affichage images d'origine bidimensionnelles — 5008

Sauvegarde) Données Relecture ) — 5009

IV